# FASCICULE DE BREVET EUROPEEN

(11) **EP 3 316 895 B1**
(45) Date de publication et mention de la délivrance du brevet: **18.09.2019**
(21) Numéro de dépôt: 16744446.2
(22) Date de dépôt: 30.06.2016
(51) Int. Cl.: A01N 1/02, A61K 38/38, A61K 35/15, A61K 35/17, A61K 35/33, A61K 35/34, A61K 35/12

(54) **PROCÉDÉ DE CRYOCONSERVATION DE CELLULES À VISÉE THÉRAPEUTIQUE**
VERFAHREN ZUR KRYOKONSERVIERUNG VON ZELLEN FÜR THERAPEUTISCHE ZWECKE
METHOD FOR THE CRYOPRESERVATION OF CELLS FOR THERAPEUTIC PURPOSES

(30) Priorité: 30.06.2015 FR 1556169
(43) Date de publication de la demande: 09.05.2018
(73) Titulaire: Laboratoire Français du Fractionnement et des Biotechnologies, 91940 Les Ulis (FR)
(72) Inventeur: DE LARICHAUDY, Joffrey, 92240 Malakoff (FR)
(74) Mandataire: Cabinet Plasseraud
(86) Numéro de dépôt international: PCT/FR2016/051630
(87) Numéro de publication internationale: WO 2017/001783

(56) Documents cités:
- US-A1- 2005 026 133
- JACK Y.J. HUANG ET AL: "Combining ovarian tissue cryobanking with retrieval of immature oocytes followed by in vitro maturation and vitrification: an additional strategy of fertility preservation", FERTILITY AND STERILITY., vol. 89, no. 3, 1 mars 2008 (2008-03-01), pages 567-572, XP055267368, USA ISSN: 0015-0282, DOI: 10.1016/j.fertnstert.2007.03.090 & Site internet de la société ORIGIO: "For Efficient Embryo Cryopreservation MediCult Vitrification Media @BULLET Simple and easy-to-follow protocol @BULLET Ready-to-use media @BULLET DMSO free 1228 4001 MediCult Vitrification Cooling 1229 5002 MediCult Vitrification Warming", , 2 décembre 2010 (2010-12-02), XP055267367, Extrait de l'Internet: URL:http://ordering.origio.com/~/media/Ori gio/Files/PDS/MEC/Vitrification_FDA_Dec201 0_v1_web_2.ashx [extrait le 2016-04-21]
- BISSOYI AKALABYA ET AL: "Effects of non-toxic cryoprotective agents on the viability of cord blood derived MNCs", CRYO LETTERS, CRYOLETTERS LLP, UK, vol. 34, no. 5, 1 septembre 2013 (2013-09-01), pages 453-465, XP009186819, ISSN: 0143-2044
- DAVID BERZ ET AL: "Cryopreservation of hematopoietic stem cells", AMERICAN JOURNAL OF HEMATOLOGY, vol. 82, no. 6, 1 juin 2007 (2007-06-01), pages 463-472, XP055107084, ISSN: 0361-8609, DOI: 10.1002/ajh.20707
- YAN LI ET AL: "Bioprocessing of Cryopreservation for Large-Scale Banking of Human Pluripotent Stem Cells", BIORESEARCH OPEN ACCESS, vol. 1, no. 5, 1 octobre 2012 (2012-10-01) , pages 205-214, XP055231320, ISSN: 2164-7860, DOI: 10.1089/biores.2012.0224

## Description

La présente invention se rapporte à une composition de cryoconservation comprenant, dans un milieu physiologiquement acceptable :
a) de la sérum albumine humaine,
b) au moins un saccharide choisi parmi les disaccharides et les trisaccharides,
c) du coenzyme Q10, et au moins un alcanediol en C3-C5, et
d) des cellules à visée thérapeutique, à l'exception de lymphocytes infiltrant la tumeur spécifiques.

La présente invention se rapporte également à un procédé de cryoconservation d'au moins un échantillon de cellules à visée thérapeutique, à l'exception de lymphocytes infiltrant la tumeur spécifiques, comprenant les étapes suivantes :
i) mélange de l'échantillon de cellules à visée thérapeutique avec :
   a) de la sérum albumine humaine,
   b) au moins un saccharide choisi parmi les disaccharides et les trisaccharides, et
   c) du coenzyme Q10, et au moins un alcanediol en C3-C5, puis
ii) congélation du mélange obtenu à l'étape i),
   lesdits lymphocytes infiltrant la tumeur étant obtenus par culture *in vitro* de lymphocytes infiltrant la tumeur à partir de prélèvement de nodules cutanés en transit, ganglion ou métastase d'un patient atteint d'un mélanome de stade 3 ou 4, ladite culture comprenant l'émergence desdits lymphocytes infiltrant la tumeur contenus dans le prélèvement de nodules cutanés en transit, ganglion ou métastase, puis la stimulation des lymphocytes infiltrant la tumeur issus de l'étape d'émergence, puis enfin l'amplification des lymphocytes infiltrant la tumeur stimulés.

La cryoconservation est un processus dans lequel des échantillons biologiques sont stockés à basse température. La cryoconservation de matériel biologique est généralement effectuée par la congélation dudit matériel, dans un support approprié, tel qu'un tube ou une ampoule en verre ou en matière plastique (généralement appelé "paillette" ou tube de congélation dans le domaine de la cryoconservation), ledit support étant adapté pour le stockage à long terme et à basse température.

La cryoconservation pose cependant un certain nombre de problèmes et de contraintes techniques. Notamment, des lésions cellulaires peuvent se produire lors de la décongélation, entraînant l'apoptose ou l'éclatement des cellules. En outre, la survie des cellules cryoconservées peut dépendre des conditions et des techniques employées lors de la congélation. Le contrôle de la vitesse de refroidissement est important : un refroidissement à faible vitesse permet une cristallisation ordonnée de l'eau congelable à l'extérieur des cellules - les cellules se déshydratent, se rétrécissent et l'eau sort de la cellule. Dans le cas contraire, la formation de glace intracellulaire entraîne la destruction des structures membranaires qui est létale pour la cellule.

Pour la plupart des cellules de mammifères, comme cela est le cas pour les produits et médicaments de thérapie cellulaire, que les cellules soient ou non génétiquement modifiées, il est en outre indispensable d'utiliser des cryoprotectants pour préserver l'intégrité et la fonctionnalité cellulaires.

Actuellement, la fabrication à une échelle industrielle (européenne ou mondiale notamment) de produits de thérapie cellulaire pose de nouvelles problématiques, telles que la stabilité du produit fini administré et la variabilité liée à la matière biologique de départ.

Dans la plupart des cas, le produit fini est conditionné :
- frais dans un milieu liquide (type albumine ou solution saline) avec une conservation limitée à quelques heures ou jours, ou bien
- congelé dans une formulation simple à base de DMSO, peu stable et peu efficace à long terme. La quantité non négligeable de cellules mortes, de débris aux effets potentiellement immunogènes et la toxicité pour le patient des excipients couramment utilisés sont autant de limites. La plupart du temps, un lavage des cellules pour retirer ces éléments toxiques (biologiques ou non) est indiqué : ces solutions ne sont pas compatibles avec une distribution industrielle. De plus, elles offrent peu de flexibilité d'administration et de stockage, au contraire des autres classes de médicaments « classiques ».

Il existe donc un besoin pour le développement d'un produit et/ou médicament de thérapie cellulaire qui soit stable à long terme (i.e. pendant plusieurs mois), qui soit facile à utiliser, directement injectable et non toxique.

La présente invention permet de répondre à ce besoin. En effet, la composition selon l'invention permet d'obtenir des produits de thérapie cellulaire comprenant des cellules à visée thérapeutique, prêts à l'emploi (i.e. prêts à être injectés sans lavage, ce qui évite toutes manipulations supplémentaires provoquant une baisse de viabilité et une perte de cellules), stables à long terme, faciles à utiliser et non toxiques.

La présente invention se rapporte donc à une composition comprenant, dans un milieu physiologiquement acceptable :
a) de l'albumine humaine (ou sérum albumine humaine),
b) au moins un saccharide choisi parmi les disaccharides et les trisaccharides,
c) du coenzyme Q10, et au moins un alcanediol en C3-C5, et
d) des cellules à visée thérapeutique, à l'exception de lymphocytes infiltrant la tumeur,
   lesdits lymphocytes infiltrant la tumeur étant obtenus par culture *in vitro* de lymphocytes infiltrant la tumeur à partir de prélèvement de nodules cutanés en transit, ganglion ou métastase d'un patient atteint d'un mélanome de stade 3 ou 4, ladite culture comprenant l'émergence (appelée également la « sortie » ou la « culture primaire ») desdits lymphocytes infiltrant la tumeur contenus dans le prélèvement de nodules cutanés en transit, ganglion ou métastase, puis la stimulation des lymphocytes infiltrant la tumeur issus de l'étape d'émergence, puis enfin l'amplification des lymphocytes infiltrant la tumeur stimulés.

Ces lymphocytes infiltrant la tumeur (appelés également « TILs » pour « Tumor Infiltrating Lymphocytes ») sont obtenus par culture *in vitro* de lymphocytes infiltrant la tumeur à partir de prélèvement de nodules cutanés en transit, ganglion ou métastase d'un patient atteint d'un mélanome de stade 3 ou 4, ladite culture comprenant :
- l'émergence desdits lymphocytes infiltrant la tumeur contenus dans le prélèvement de nodules cutanés en transit, ganglion ou métastase. Cette émergence est également appelée « culture primaire » ou « sortie » ; c'est l'étape au cours de laquelle les lymphocytes infiltrant la tumeur, initialement contenus dans le prélèvement de nodules cutanés en transit, ganglion ou métastase, passent dans le milieu de culture et sont cultivés *in vitro,* puis
- la stimulation des lymphocytes infiltrant la tumeur issus de l'étape d'émergence, puis enfin
- l'amplification des lymphocytes infiltrant la tumeur stimulés.

De tels TILs sont appelés « lymphocytes infiltrant la tumeur spécifiques » ou « lymphocytes spécifiques infiltrant la tumeur » dans la présente demande.

La présente invention se rapporte également à un procédé de cryoconservation d'au moins un échantillon de cellules à visée thérapeutique, à l'exception de lymphocytes infiltrant la tumeur spécifiques, comprenant les étapes suivantes :
i) mélange de l'échantillon de cellules à visée thérapeutique avec :
   a) d'abord de l'albumine humaine, puis
   b) au moins un saccharide choisi parmi les disaccharides et les trisaccharides, et c) du coenzyme Q10, et au moins un alcanediol en C3-C5, puis
ii) congélation du mélange obtenu à l'étape i),
   lesdits lymphocytes infiltrant la tumeur étant obtenus par culture *in vitro* de lymphocytes infiltrant la tumeur à partir de prélèvement de nodules cutanés en transit, ganglion ou métastase d'un patient atteint d'un mélanome de stade 3 ou 4, ladite culture comprenant l'émergence desdits lymphocytes infiltrant la tumeur contenus dans le prélèvement de nodules cutanés en transit, ganglion ou métastase, puis la stimulation des lymphocytes infiltrant la tumeur issus de l'étape d'émergence, puis enfin l'amplification des lymphocytes infiltrant la tumeur stimulés.

La présente invention se rapporte également à l'utilisation d'une composition comprenant, dans un milieu physiologiquement acceptable :
a) de l'albumine humaine,
b) au moins un saccharide choisi parmi les disaccharides et les trisaccharides, et
c) du coenzyme Q10, et au moins un alcanediol en C3-C5, pour la cryoconservation d'au moins un échantillon de cellules à visée thérapeutique, à l'exception de lymphocytes infiltrant la tumeur spécifiques ,
   lesdits lymphocytes infiltrant la tumeur étant obtenus par culture *in vitro* de lymphocytes infiltrant la tumeur à partir de prélèvement de nodules cutanés en transit, ganglion ou métastase d'un patient atteint d'un mélanome de stade 3 ou 4, ladite culture comprenant l'émergence desdits lymphocytes infiltrant la tumeur contenus dans le prélèvement de nodules cutanés en transit, ganglion ou métastase, puis la stimulation des lymphocytes infiltrant la tumeur issus de l'étape d'émergence, puis enfin l'amplification des lymphocytes infiltrant la tumeur stimulés.

Dans la présente demande, les lymphocytes infiltrant la tumeur spécifiques exclus de la composition selon l'invention sont autologues ; ils correspondent aux lymphocytes infiltrant la tumeur contenus dans la(es) tumeur(s).

Ces lymphocytes infiltrant la tumeur exclus de la présente invention sont obtenus par culture *in vitro* de lymphocytes infiltrant la tumeur à partir de prélèvement de nodules cutanés en transit, ganglion ou métastase d'un patient atteint d'un mélanome de stade 3 ou 4, ladite culture comprenant :
- l'émergence desdits lymphocytes infiltrant la tumeur contenus dans le prélèvement de nodules cutanés en transit, ganglion ou métastase. Cette émergence est également appelée « culture primaire » ou « sortie » ; c'est l'étape au cours de laquelle les lymphocytes infiltrant la tumeur, initialement contenus dans le prélèvement de nodules cutanés en transit, ganglion ou métastase, passent dans le milieu de culture et sont cultivés *in vitro,* puis
- la stimulation des lymphocytes infiltrant la tumeur issus de l'étape d'émergence, puis enfin
- l'amplification des lymphocytes infiltrant la tumeur stimulés.

En particulier, l'étape d'émergence des TILs peut être réalisée par culture primaire du prélèvement de nodules cutanés en transit, ganglion ou métastase, notamment par culture primaire dans un milieu de culture sélectif sans sérum de type X-VIVO 15® (commercialisé par Cambrex Corp) supplémenté en interleukine-2 (IL-2).

Après émergence des TILs, ces derniers subissent une étape de stimulation, de préférence dans un récipient de culture clos compartimenté, notamment en présence au moins de cellules nourricières allogéniques irradiées non proliférantes. Par « récipient de culture clos », on entend un système permettant d'entretenir des cellules en culture dans un milieu adapté, sans que celles-ci ne soient directement au contact avec l'environnement extérieur. Enfin, l'étape de stimulation des TILs est suivie d'une étape d'amplification.

De préférence, tous les TILs sont exclus des cellules à visée thérapeutique de la présente demande.

La composition selon l'invention comprend donc, dans un milieu physiologiquement acceptable :
a) de l'albumine humaine,
b) au moins un saccharide choisi parmi les disaccharides et les trisaccharides,
c) du coenzyme Q10, et au moins un alcanediol en C3-C5, et
d) des cellules à visée thérapeutique, à l'exception de lymphocytes infiltrant la tumeur spécifiques,
   lesdits lymphocytes infiltrant la tumeur étant obtenus par culture *in vitro* de lymphocytes infiltrant la tumeur à partir de prélèvement de nodules cutanés en transit, ganglion ou métastase d'un patient atteint d'un mélanome de stade 3 ou 4, ladite culture comprenant l'émergence desdits lymphocytes infiltrant la tumeur contenus dans le prélèvement de nodules cutanés en transit, ganglion ou métastase, puis la stimulation des lymphocytes infiltrant la tumeur issus de l'étape d'émergence, puis enfin l'amplification des lymphocytes infiltrant la tumeur stimulés.

De préférence, la composition selon l'invention est substantiellement exempte de diméthylsulfoxyde (DMSO). Par « substantiellement exempte », on entend que la composition contient moins de 3% en poids de DMSO, de préférence moins de 1% en poids. De préférence, la composition selon l'invention est totalement dépourvue de DMSO.

Par « milieu physiologiquement acceptable », on entend un milieu aqueux comprenant des électrolytes. Les électrolytes sont par exemple des sels de sodium, de potassium, de magnésium et/ou de calcium avec des anions de type chlorure, carbonate, hydroxyde ou caprylate. De préférence, le milieu physiologiquement acceptable est un milieu aqueux comprenant du chlorure de sodium et du caprylate de sodium.

La composition selon l'invention comprend la sérum albumine humaine (ou albumine humaine, composé a)). Cette protéine est une protéine plasmatique de haut poids moléculaire (environ 65 kDa). C'est la protéine la plus abondante du plasma ; sa concentration moyenne normale est de 38 à 48 g/l. Elle permet de tamponner le pH et de maintenir l'osmolarité. Sa pureté est de préférence de 95%. On peut également utiliser un ou plusieurs fragments et/ou dérivés de la sérum albumine humaine, lesdits fragments ou dérivés étant non immunogènes et ayant une propriété oncotique similaire à la sérum albumine humaine.

De préférence, la sérum albumine humaine, ses fragments et/ou dérivés, est présente en quantité comprise entre 2 et 10% en poids par rapport au poids total de composition, de préférence entre 2.5 et 6% en poids, de préférence entre 3.5 et 4.5% en poids.

Dans la présente demande, sauf mention contraire, les quantités sont mentionnées en poids par rapport au poids total de composition.

La composition selon l'invention comprend également au moins un saccharide (composé b)). Le saccharide améliore la survie et la fonction des cellules en préservant l'équilibre osmotique. Une fraction pénètre les cellules et permet de stabiliser les structures membranaires. Le saccharide est choisi parmi les disaccharides et les trisaccharides.

Le disaccharide a de préférence pour formule A-B, dans laquelle A et B sont chacun indépendamment choisis parmi le glucose, le fructose et le mannose. Le saccharide est de préférence un disaccharide. Le disaccharide est de préférence un dimère de glucose. Plus préférentiellement, le disaccharide est choisi parmi le tréhalose et le saccharose. Plus préférentiellement, le disaccharide est le tréhalose.

Les trisaccharides sont de préférence choisis parmi le raffinose (trimère de galactose, glucose et fructose), le maltotriose et l'isomaltotriose (trimères de glucose).

Le saccharide est de préférence présent dans la composition selon l'invention en concentration comprise entre 0.05M et 1M, de préférence entre 0.07M et 0.5M, de préférence entre 0.08M et 0.12M.

La composition selon l'invention comprend enfin au moins du coenzyme Q10 ou de la L-cystéine, et au moins un alcanediol en C3-C5 (composés c)). La composition selon l'invention comprend ainsi, comme composés c), au moins du coenzyme Q10 et au moins un alcanediol en C3-C5.

Le coenzyme Q10, également appelé ubiquinone, est un composé chimique comprenant un groupement quinone. Son nom chimique est le 2,3-diméthoxy-5-méthyl-6-décaprénylbenzoquinone. Il est de préférence présent dans la composition selon l'invention en quantité comprise entre 0.005 et 1% en poids, de préférence entre 0.007 et 0.5% en poids, de préférence entre 0.007 et 0.1% en poids.

L'alcanediol en C3-C5 est de préférence un alcane linéaire, ramifié ou cyclique comprenant de 3 à 5 atomes de carbone, et 2 groupements hydroxyl. De préférence, il est choisi parmi les alcanes linéaires comprenant de 3 à 5 atomes de carbone, et 2 groupements hydroxyl. Plus préférentiellement, il est choisi parmi le propane-1,2-diol, le pentane-1,5-diol et le butane-2,3-diol. L'alcanediol en C3-C5 est de préférence le propane-1,2-diol (également appelé propylène glycol).

L'alcanediol en C3-C5 est de préférence présent dans la composition selon l'invention en quantité comprise entre 3 et 10% en volume, de préférence entre 3 et 7% en volume.

De préférence, la composition selon l'invention comprend, dans un milieu physiologiquement acceptable :
a) de l'albumine humaine, de préférence en quantité comprise entre 2.5 et 6% en poids,
b) un saccharide, de préférence du tréhalose, de préférence en concentration comprise entre 0.07M et 0.5M, et
c) du coenzyme Q10 et du propane-1,2-diol, de préférence respectivement en quantité comprise entre 0.007 et 0.1% en poids, et en quantité comprise entre 3 et 10% en volume.

La composition selon l'invention est particulièrement intéressante, et vise à cryoconserver au moins un échantillon de cellules à visée thérapeutique. En effet, les composés a) à c) utilisés dans la composition selon l'invention permettent de cryoconserver les cellules à visée thérapeutique, de façon durable et efficace.

Les cellules à visée thérapeutique (composés d)) sont de préférence choisies parmi :
- les cellules immunitaires, telles que les cellules NK, les monocytes, les lymphocytes B, les lymphocytes T, naturels ou génétiquement modifiés, tels que les lymphocytes T régulateurs, les lymphocytes T cytotoxiques, les lymphocytes T auxiliaires (ou helper) et les lymphocytes T ayant un récepteur antigénique chimérique (CAR) ;
- les myoblastes humains ;
- les cellules souches hématopoïétiques ;
- les cellules souches mésenchymateuses ;
- les cellules cardiaques ;
- les fibroblastes ; et
- toutes autres cellules naturelles ou génétiquement modifiées.

Les cellules NK (ou lymphocytes NK) sont des cellules de l'immunité innée. Ce sont des lymphocytes non T (CD3-) non B (CD19-), caractérisés chez l'homme par les marqueurs CD56, CD16 et NK.

Les monocytes sont des leucocytes qui évoluent en macrophages, cellules dendritiques ou ostéoclastes.

Les lymphocytes B sont les cellules immunitaires responsables de la production d'anticorps. Les lymphocytes T régulateurs sont une sous-population de lymphocytes T CD4+, qui inhibent la prolifération d'autres lymphocytes T effecteurs.

Les lymphocytes T cytotoxiques sont une sous-population de lymphocytes T CD8+, qui détruisent les cellules infectées.

Les lymphocytes T auxiliaires (helper) sont une sous-population de lymphocytes T CD4+, intermédiaires de la réponse immunitaire.

Enfin, les lymphocytes T ayant un récepteur antigénique chimérique (CAR), appelés également cellules CAR-T, correspondent à une technologie particulière d'ingénierie cellulaire. Ce sont des lymphocytes T qui expriment un récepteur antigénique chimérique. Les cellules CAR-T sont capables de tuer les cellules cancéreuses, par reconnaissance et liaison à l'antigène tumoral présent sur lesdites cellules cancéreuses.

L'échantillon de cellules à visée thérapeutique peut provenir du patient à traiter (dans ce cas le patient et le donneur sont la même personne), par biopsie ou prélèvement sanguin. Dans ce cas, la composition obtenue, une fois cryoconservée puis décongelée, sera administrée à ce même patient : c'est un produit autologue.

Alternativement, l'échantillon de cellules à visée thérapeutique peut provenir d'une autre source (i.e. autre individu, ingénierie cellulaire), notamment par biopsie ou prélèvement sanguin. Dans ce cas, la composition obtenue, une fois cryoconservée puis décongelée, sera administrée à un patient à traiter autre que le donneur : c'est un produit allogénique.

La présente invention se rapporte également à un procédé de cryoconservation d'au moins un échantillon de cellules à visée thérapeutique, à l'exception de lymphocytes infiltrant la tumeur spécifiques, comprenant les étapes suivantes :
i) mélange de l'échantillon de cellules à visée thérapeutique avec :
   a) de l'albumine humaine,
   b) au moins un saccharide choisi parmi les disaccharides et les trisaccharides, et
   c) du coenzyme Q10, et au moins un alcanediol en C3-C5, puis
ii) congélation du mélange obtenu à l'étape i),
   lesdits lymphocytes infiltrant la tumeur étant obtenus par culture *in vitro* de lymphocytes infiltrant la tumeur à partir de prélèvement de nodules cutanés en transit, ganglion ou métastase d'un patient atteint d'un mélanome de stade 3 ou 4, ladite culture comprenant l'émergence desdits lymphocytes infiltrant la tumeur contenus dans le prélèvement de nodules cutanés en transit, ganglion ou métastase, puis la stimulation des lymphocytes infiltrant la tumeur issus de l'étape d'émergence, puis enfin l'amplification des lymphocytes infiltrant la tumeur stimulés.

Dans ce procédé, l'étape de mélange i) de l'échantillon de cellules à visée thérapeutique avec les composés a) à c) décrits ci-dessus se fait typiquement par dilution. De préférence, les cellules sont reprises dans le composé a) sous forme liquide, de préférence pour 50% du volume, puis les composés b) et c), de préférence précédemment mélangés entre eux pour obtenir une solution de concentration 2x, sont ajoutés. Le mélange de l'étape i) peut se faire aux alentours de 4°C, ou à température ambiante (i.e. 20°C).

De préférence, l'échantillon de cellules à visée thérapeutique est, quant à lui, préalablement cultivé *in vitro,* dans un milieu de culture approprié. Puis il subit une centrifugation, le surnageant est retiré et le culot est suspendu dans les composés a), puis b) et c) décrits ci-dessus.

L'étape de congélation (étape ii)) est de préférence effectuée sur une descente en température de +4°C ou température ambiante jusqu'à une température comprise entre -100°C et -160°C. L'étape de congélation (étape ii)) est de préférence effectuée jusqu'à une température comprise entre -100°C et -180°C, de préférence comprise entre -140°C et -160°C.

L'échantillon est ensuite stocké à une température inférieure à -130°C en général.

De préférence, la congélation ii) est réalisée en plaçant le mélange obtenu dans l'étape i) dans un récipient immergé dans un mélange d'isopropyl alcool à +4°C, le tout étant mis à une température comprise entre -70°C et -100°C. Ce système (« congélation en boîte Nalgène ») permet, grâce au refroidissement lent de l'alcool, une descente en température quasi-linéaire comprise entre -1°C et -2°C par minute. Alternativement, de préférence, la congélation ii) est effectuée à l'aide d'un congélateur programmé. De tels congélateurs sont notamment commercialisés par Air Liquide ou Cryobiosystem.

De préférence, la congélation ii) se fait, notamment à l'aide d'un congélateur programmé, par les étapes suivantes :
- placer le mélange obtenu à l'étape i) à une température de +4°C ; puis
- diminuer la température de 1°C par minute, de +4°C à -40°C ; puis
- diminuer la température de 10°C par minute, de -40°C à -150°C, pour atteindre une température finale de stockage d'environ -150°C.

Le produit ainsi obtenu, congelé, peut être conservé pendant quelques mois à -150°C environ. Ces températures sont celles appliquées à l'échantillon.

une descente en température quasi-linéaire comprise entre -1°C et -2°C par minute. Alternativement, de préférence, la congélation ii) est effectuée à l'aide d'un congélateur programmé. De tels congélateurs sont notamment commercialisés par Air Liquide ou Cryobiosystem.

De préférence, la congélation ii) se fait, notamment à l'aide d'un congélateur programmé, par les étapes suivantes :
- placer le mélange obtenu à l'étape i) à une température de +4°C ; puis
- diminuer la température de 1°C par minute, de +4°C à -40°C ; puis
- diminuer la température de 10°C par minute, de -40°C à -150°C, pour atteindre une température finale de stockage d'environ -150°C.

Le produit ainsi obtenu, congelé, peut être conservé pendant quelques mois à -150°C environ. Ces températures sont celles appliquées à l'échantillon.

L'invention est illustrée par les exemples suivants, nullement limitatifs.

### Exemple 1 : Tests avec la formulation selon l'invention pour cryopréserver des myoblastes

La formulation suivante, nommée PG 5%, a été préparée :
Propylène glycol 5% + Coenzyme Q10 0,01% + Tréhalose 0,1M + sérum albumine humaine (AH) 4%.

Elle nécessite un temps d'incubation de 30 minutes maximum à 4°C avant congélation.

Cette formulation complexe est associée à un cycle de descente en température automatisé effectué au moyen d'un CRF (congélateur programmé) qui permet une descente en température complexe selon un cycle défini. Jusqu'à -40°C, les cellules sont considérées comme sensibles : la descente en température est lente et progressive pour permettre la formation de cristaux réguliers. Au-dessous de ce palier, le produit est considéré comme stable et la descente en température est rapide jusqu'à la température de stockage de -150°C maximum (azote gazeux ou liquide).

### Contexte de l'étude

Au cours de l'étude de congélation des myoblastes, 4 cycles ont été effectués, dont les caractéristiques sont présentées dans le tableau suivant :

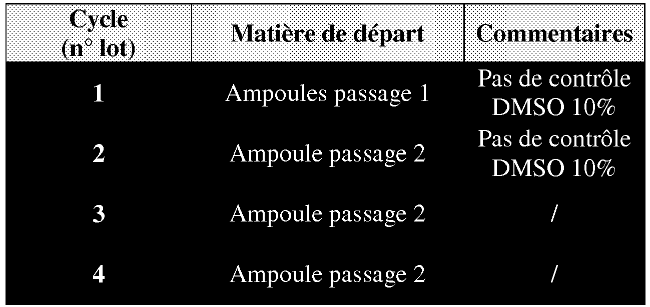

Pour ces 4 cycles, des ampoules de myoblastes issus de patients sains ont été décongelées. Les cellules ont été amplifiées puis congelées à la concentration de 5 x 10⁶ cellules/mL par CRF.

Afin de démontrer l'efficacité des formulations de congélation, 3 bras ont été réalisés. Pour chaque bras, 2 ampoules de 1mL ont été décongelées pour test.

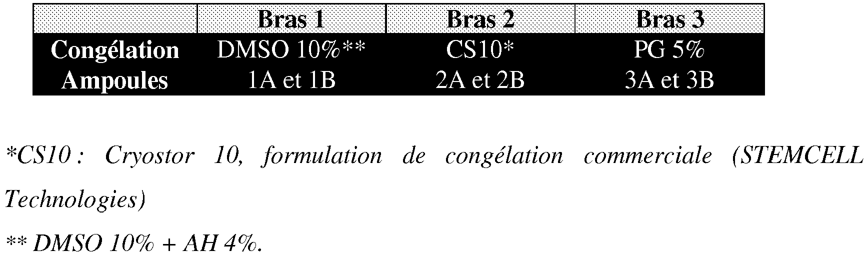

### Critère d'évaluation

Dans le but d'évaluer l'efficacité des formulations de congélation, différents tests, présentés dans le tableau suivant, ont été réalisés avant congélation (appelé TO) et après décongélation. Les résultats obtenus avec les cellules décongelées ont été comparés au TO et exprimé en % de TO. Cette première analyse permet de statuer sur l'effet de la congélation sur les cellules pour chaque bras testé.

| **T0** | **Post décongélation** | **Objectif** |
|---|---|---|
| Viabilité | Viabilité à 0h post décongélation | Critère général (1^{er} critère observé souvent libératoire qui permet ou non l'injection corrélé à l'efficacité et aux effets secondaires (augmentation des impuretés acellulaires dues aux débris, agrégats,...) |
| | Viabilité à 4h post décongélation | |
| Phénotype | | Caractérisation des sous-populations cellulaires et en particulier des cellules d'intérêt. En effet, le cycle de congélation/décongélation peut affecter spécifiquement certains sous types plus sensibles et modifier la balance du produit injecté (augmentation des impuretés cellulaires) |
| Test de prolifération | | Détermination du facteur d'amplification des cellules et donc de leur capacité de reprise corrélée à leur activité métabolique/fonctionnelle |

Les différents bras ont été comparés par une analyse statistique. La formulation de référence (bras 1) est le DMSO 10% + AH 4% (formulation basique très répandue dans les centres d'injection de cellules hématopoïétiques). Pour les cycles 1 et 2, le bras 2 (congélation en CS10) a servi de référence en l'absence de bras DMSO 10%.

Lors de l'analyse statistique, l'homogénéité des variances a été analysée par un test de Fisher (F-Test, variance homogène si p > 0,05). Un test d'égalité des espérances avec deux observations de variances égales était fait en cas de variance homogène. Dans le cas de variance non homogène, un test d'égalité des espérances à deux observations de variances différentes était réalisé. Les moyennes sont non significativement différentes si p > 0,05. Les moyennes sont significativement différente si p < 0,05.

### Résultats

### Analyse de la viabilité des cellules

L'analyse de la viabilité *post* décongélation démontre que les conditions testées sont non significativement différentes du TO pour tous les bras (90 à 109% du TO).
Les conditions PG 5% et CS10 sont stables.

Pour le lot 4, les conditions CS10, PG5% et DMSO 10% sont non différentes entre elles et par rapport au T0.

Pour les lots 3 et 2, les 2 formulations testées sont non différentes du TO et du DMSO 10%. A noter que le bras 3 PG 5% est toujours non différent du bras CS10 quel que soient les cycles.

### Au final, les écarts sont faibles entre les différentes formulations donc la viabilité post décongélation n'est pas un critère discriminant.

### Viabilités à 0h post décongélation :

Lot 1 : 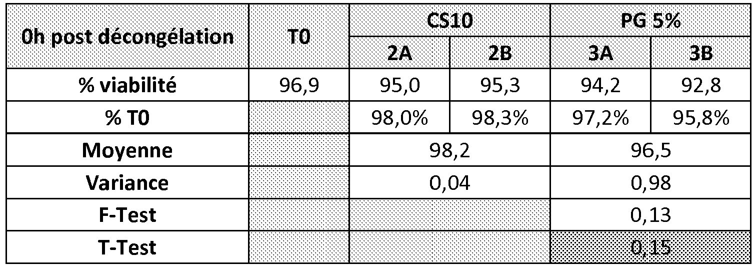
Lot 2 : 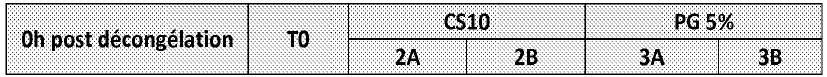 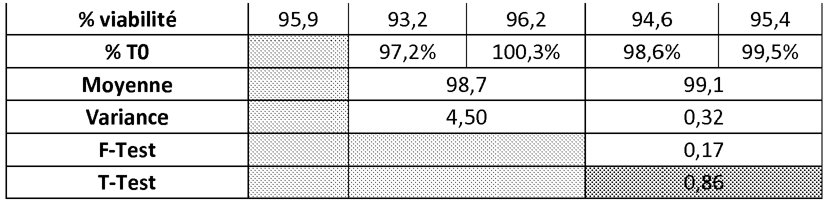
Lot 3 : 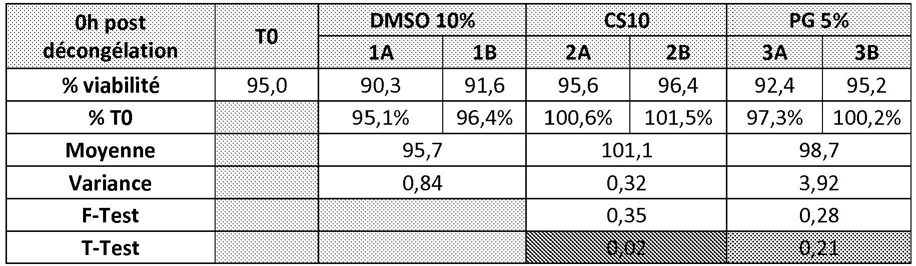
Lot 4 :

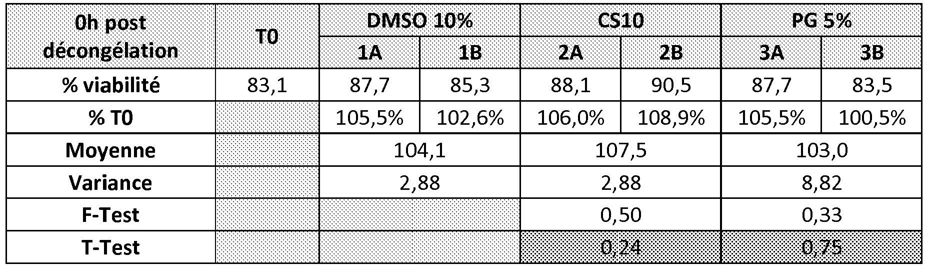

L'analyse de la viabilité des cellules après 4 heures à température ambiante dans leur contenant primaire et leur formulation de congélation permet d'évaluer la stabilité décongelée des cellules (simule les conditions de manipulation, transport et attente avant injection au patient en conditions réelles). Elle met en évidence une baisse modérée des pourcentages de viabilités pour les 3 bras, inférieure à 20 % du TO qui est acceptable.

Après analyse des formulations, dans le cas du PG 5%, il n'y a aucune différence significative par rapport au TO et au contrôle positif CS10. Cela démontre que la formulation PG5% est plus efficace pour conserver des myoblastes après 4 heures de décongélation.

### Viabilités à 4h post décongélation :

Lot 1 : 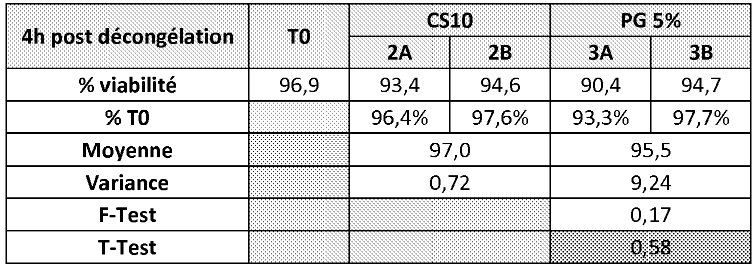
Lot 2 : 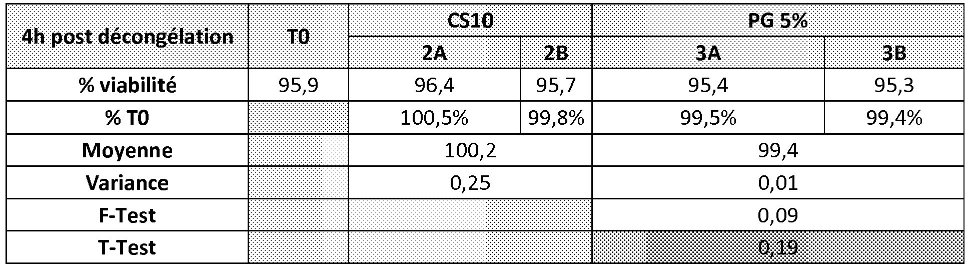
Lot 3 : 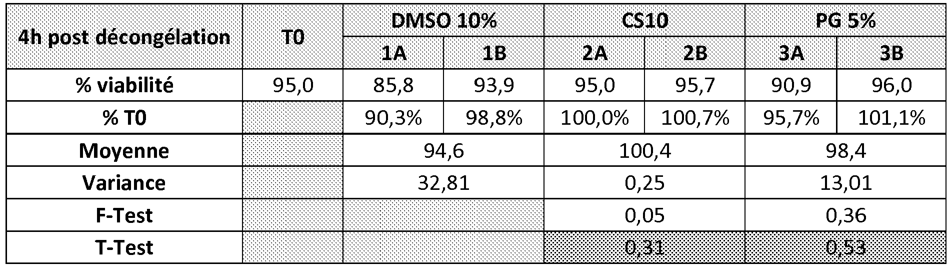
Lot 4 :

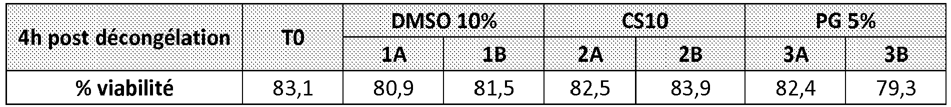

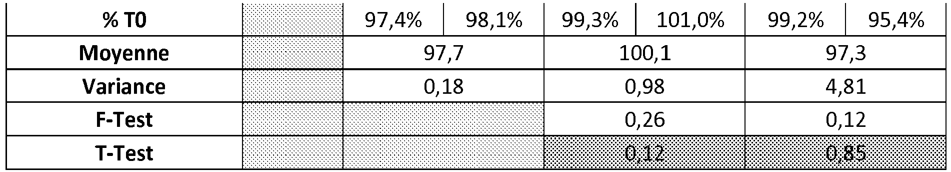

### Analyse du phénotype

Un phénotype a été effectué afin de déterminer la stabilité des myoblastes dans le produit. Pour les 3 premiers lots, les pourcentages retrouvés sont identiques au TO et non significativement différents du bras référent DMSO 10%. Pour le lot 4, le pourcentage de myoblastes diminue dans la formulation PG 5% : la baisse observée est due à des valeurs très regroupées et au nombre limité de réplicats (passe de 64,8% sur le TO à 60,7% sur PG5%).

### Phénotypes

Lot 1 : 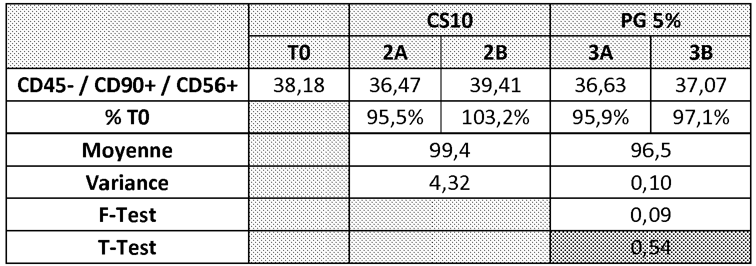
Lot 2 : 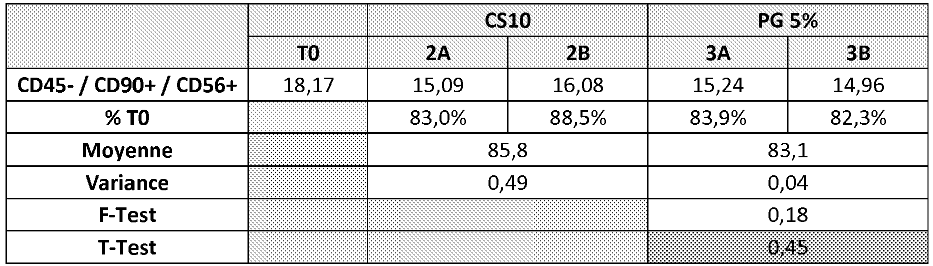
Lot 3 : 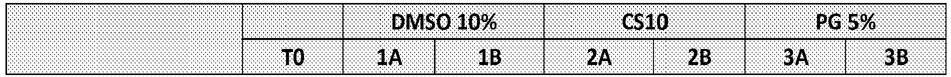 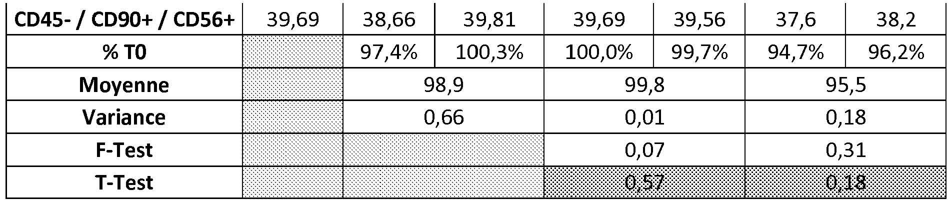
Lot 4 :

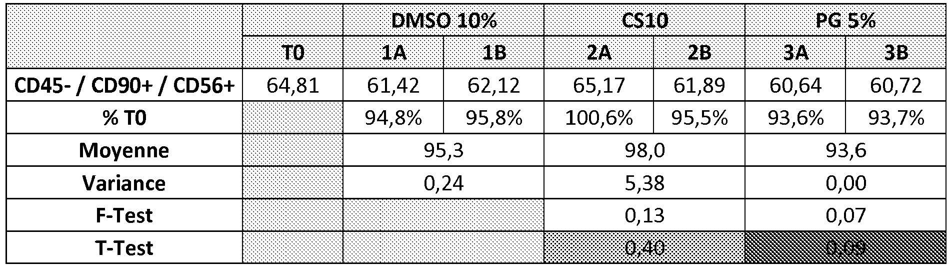

### Analyse de la prolifération des cellules

Lot 1 : 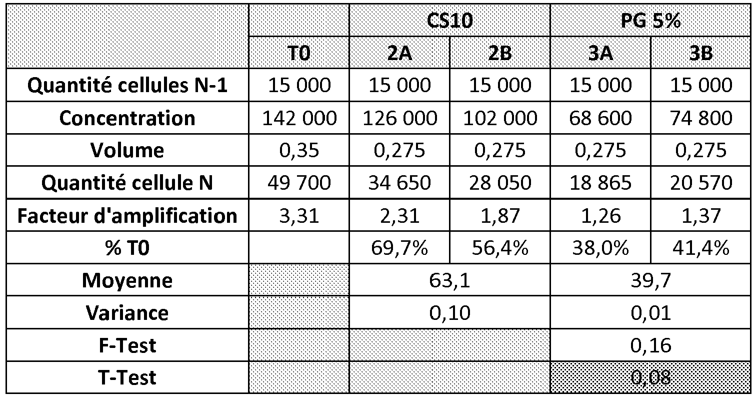
Lot 2 : 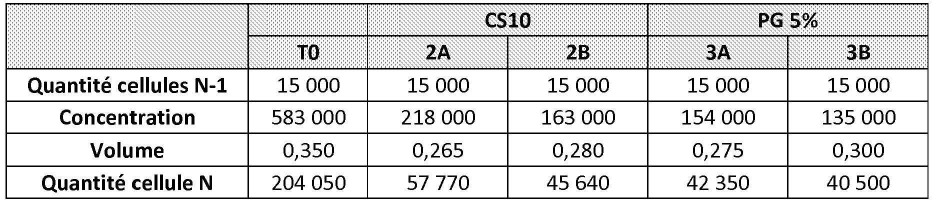 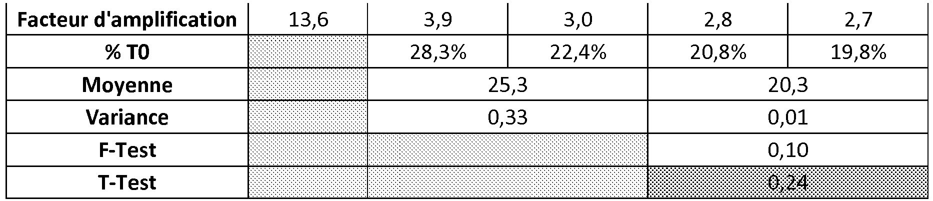
Lot 3 : 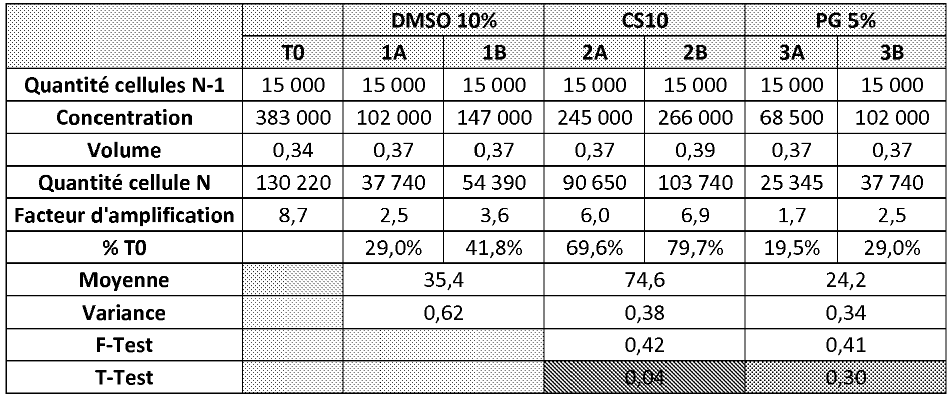
Lot 4 :

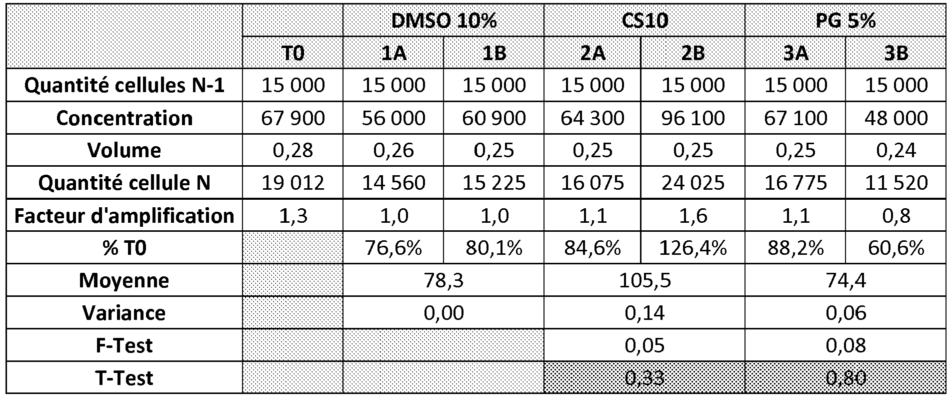

Les tests de prolifération ont été réalisés avec des temps d'incubation différents :
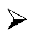 Lot 1 : 3 jours de prolifération.
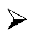 Lot 2 : 5 jours de prolifération.
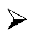 Lot 3 : 5 jours de prolifération.
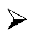 Lot 4 : 3 jours de prolifération.

Dans un premier temps, l'analyse des résultats démontrent que le potentiel de prolifération des cellules est diminué après congélation dans tous les cas. En effet, les résultats en pourcentages de TO varient fortement. Les facteurs d'amplification des cellules formulées en PG 5% ne sont pas significativement différents des bras référents.

### Conclusion de l'étude

La présente étude de congélation a donc été réalisée sur 4 lots de myoblastes humains de donneurs différents. Parmi les critères étudiés, les conclusions sont :
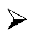 **Viabilité des cellules**. La cryo-préservation des myoblastes est efficace en formulations PG 5% en terme de viabilité cellulaire puisqu'elle est toujours supérieure à 80% du TO. L'efficacité du PG 5% est très bonne. Les résultats obtenus à 4h post décongélation sont corrects et valident une utilisation clinique.
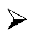 **Test de prolifération**. La congélation des myoblastes entraîne une baisse inhérente de la capacité de prolifération des cellules traduit par des facteurs d'amplification plus faible que le TO. Cette baisse est commune à tous les bras. Ce phénomène, couramment observé au laboratoire, n'est pas un critère d'exclusion de nos formulations de congélation.
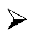 **Phénotype**. La formulation de congélation PG 5% préserve parfaitement le pourcentage de cellules d'intérêts (myoblastes).

### Exemple 2 : Tests de la formulation selon l'invention pour cryopréserver des cellules mononuclées du sang (CMN)

La formulation suivante, nommée PG 5%, a été préparée :
Propylène glycol 5% + Coenzyme Q10 0,01% + Tréhalose 0,1M + sérum albumine humaine (AH) 4%.

Elle nécessite un temps d'incubation de 30 minutes maximum à 4°C avant congélation.

Cette formulation complexe est associée à un cycle de descente en température automatisé effectué au moyen d'un CRF (congélateur programmé) qui permet une descente en température complexe selon un cycle défini. Jusqu'à -40°C, les cellules sont considérées comme sensibles : la descente en température est lente et progressive pour permettre la formation de cristaux réguliers. Au-dessous de ce palier, le produit est considéré comme stable et la descente en température est rapide jusqu'à la température de stockage de -150°C maximum (azote gazeux ou liquide).

### Contexte de l'étude

Au cours de l'étude de congélation des CMN, 2 cycles ont été effectués, dont les caractéristiques sont présentées dans le tableau suivant :

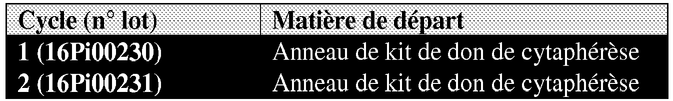

Pour ces deux cycles, des CMN ont été isolés à partir d'anneau de kit de don de cytaphérèse après ficoll. Après une incubation de 24h en flasque, permettant l'adhésion des monocytes, les CMN ont été congelés à la concentration de **20 x 10⁶ cellules/mL** par CRF.

Afin de démontrer l'efficacité des formulations de congélation, 3 bras sont réalisés. Pour chaque bras, **2 ampoules** de 1mL ont été décongelées pour test.

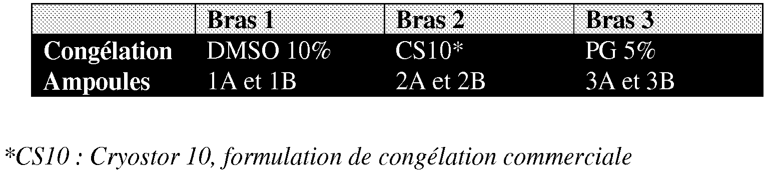

### Critère d'évaluation

Dans le but d'évaluer l'efficacité des formulations de congélation, différents tests, présentés dans le tableau suivant, ont été réalisés avant congélation (appelé TO) et après décongélation. Les résultats obtenus avec les cellules décongelées ont été comparés au TO et exprimé en % de TO. Cette première analyse permet de statuer sur l'effet de la congélation sur les cellules pour chaque bras testé.

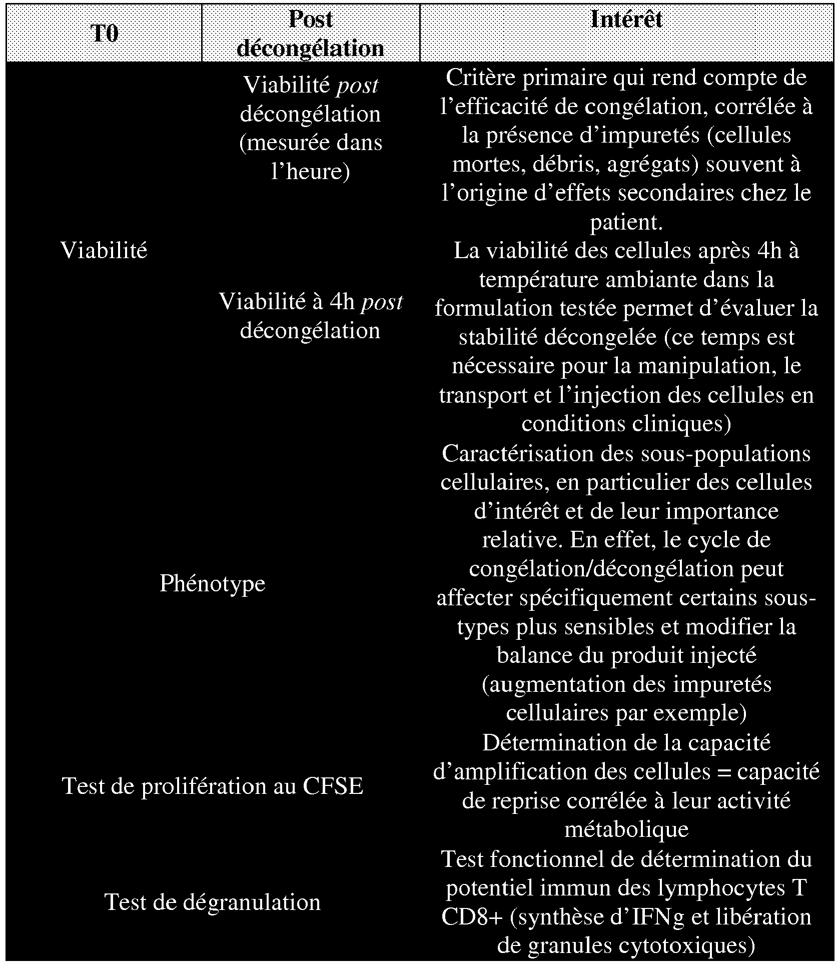

Dans un second temps, les différents bras de congélation ont été comparés par une analyse statistique. La formulation de congélation de référence couramment utilisée au sein des laboratoires étant le DMSO 10% + AH 4%, le bras 1 a servi de référence pour l'analyse statistique.

Lors de l'analyse statistique, l'homogénéité des variances a été analysée par un test de Fisher (F-Test, variance homogène si p > 0,05) et selon le résultat, un test d'égalité des espérances avec deux observations de variances égales ou différentes était réalisé. Les moyennes sont significativement différentes si p < 0,05.

### Analyse de la viabilité des cellules

Pour chaque lot, l'analyse de la viabilité *post* décongélation démontre que les pourcentages obtenus à décongélation sont équivalents au TO pour les bras 1, 2 et 3 (> 96,0% du T0). A noter que la viabilité des conditions 1 à 3 est stable par rapport au T0.

Pour le lot 16Pi00230, les bras 2 et 3 ne présentent pas de différence avec le bras 1.

Pour le lot 16Pi00231, la condition PG 5% est significativement supérieure à la condition CS10 et DMSO 10% à Oh *post* décongélation. En effet, la viabilité a tendance à augmenter par rapport au TO (104,2 %, les centrifugations ayant pu éliminer des cellules mortes / débris).

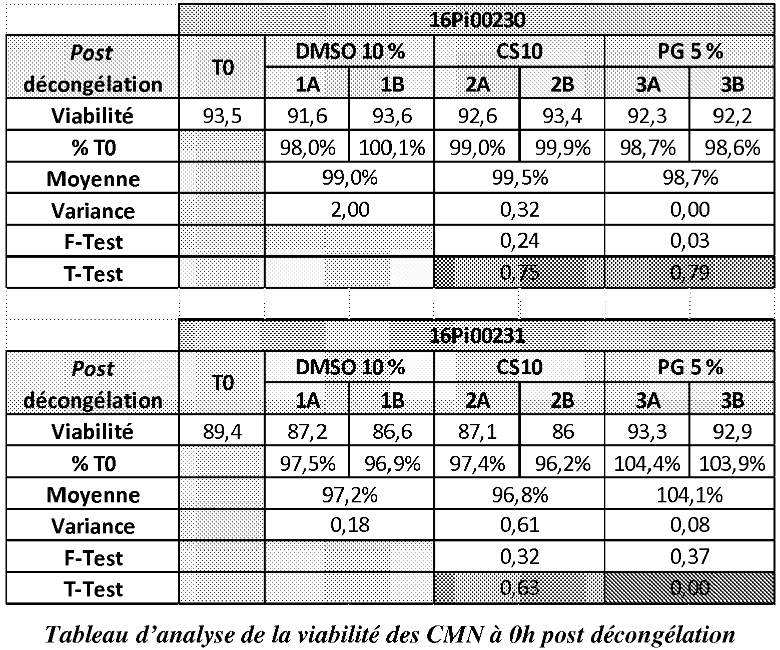

L'analyse de la viabilité à 4h *post* décongélation met en évidence une baisse modérée des pourcentages de viabilités pour les 4 bras par rapport au TO, inférieure ou égale à 12%. A ce stade, la formulation PG 5% est meilleure que la formulation DMSO 10%.

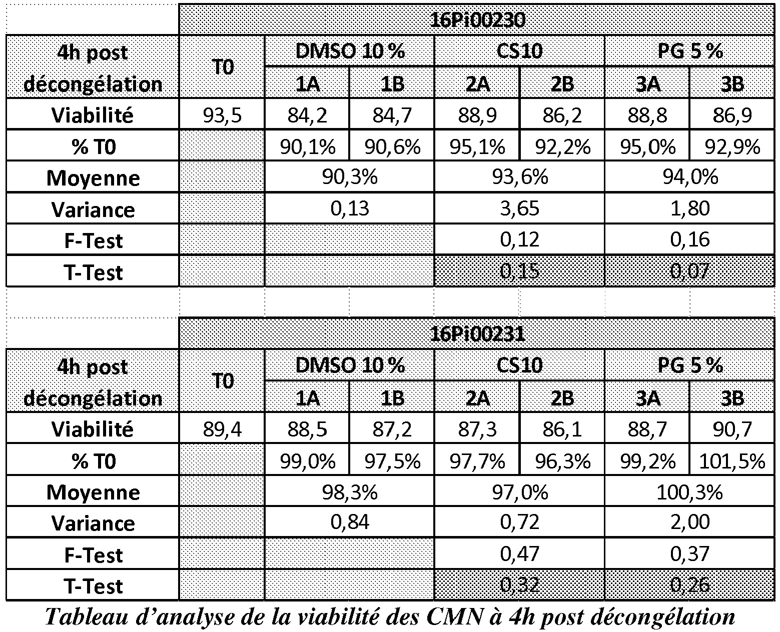

### Analyse de la prolifération des cellules par test CFSE

Cette méthode d'immuno-marquage a pour but de mesurer la prolifération des cellules en observant l'extinction d'un marquage intracellulaire, le CFSE (CarboxyFluorescein diacetate Succinimidyl Ester), qui est dilué par la division de la cellule-mère marquée en 2 cellules-filles. Il rend compte de l'état fonctionnel des cellules.

Pour chacun des lots, le test démontre la capacité des cellules décongelées à proliférer pour tous les bras. L'analyse statistique conclut à l'absence de différence significative entre les bras congelés en CS10 et PG 5% versus le DMSO 10%. Cependant, la formulation testée et le CS10 donnent des résultats plus homogènes (variance faible sur les duplicats) tandis que le contrôle DMSO 10% est très variable (écart type = 14,5% pour le lot 16Pi00230).

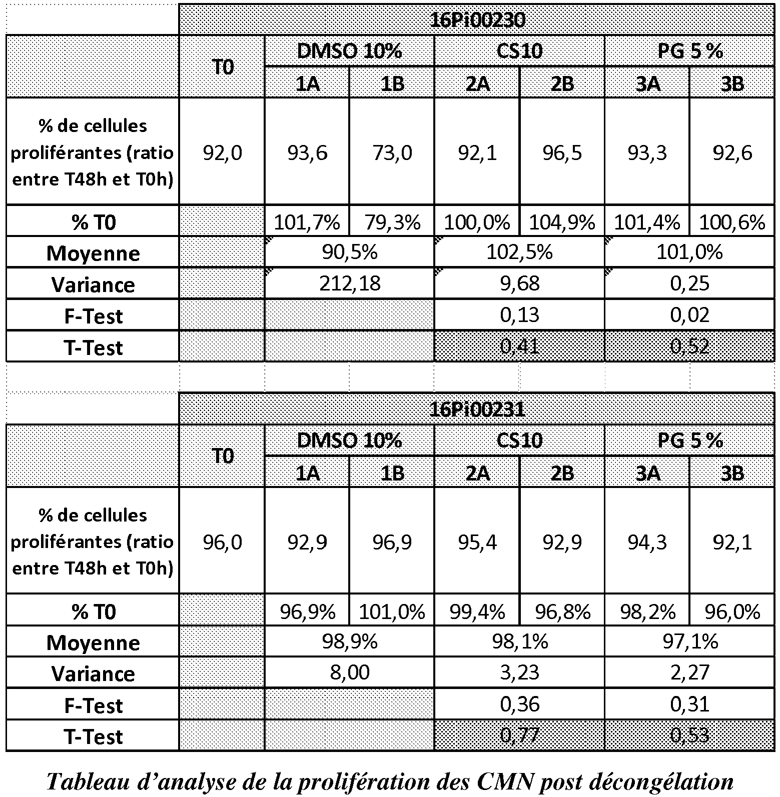

### Analyse du test de dégranulation

Le test de dégranulation permet d'évaluer la capacité immune des cellules (en particulier la population CD8+ cytotoxique effectrice) en réponse à un signal d'activation CD3-CD28 mimé par des billes.

Le pourcentage de cellules dégranulantes est diminué dans toutes les conditions congelées par rapport à T0.

Pour le lot 16Pi00230, la comparaison des différentes conditions entre elles, ne démontre pas de différence significative puisque le référent DMSO 10% est aussi très variable (=16,2 ± 6,7). Parmi les 2 autres conditions testées, le PG5% est la formulation qui préserve mieux la fonctionnalité des cellules.

Pour le lot 16Pi00231, dans les cas des conditions 1 à 3, cette baisse est modérée et acceptable. Pour les bras 1 et 2, les pourcentages de cellules proliférantes sont stables (environ 20%).

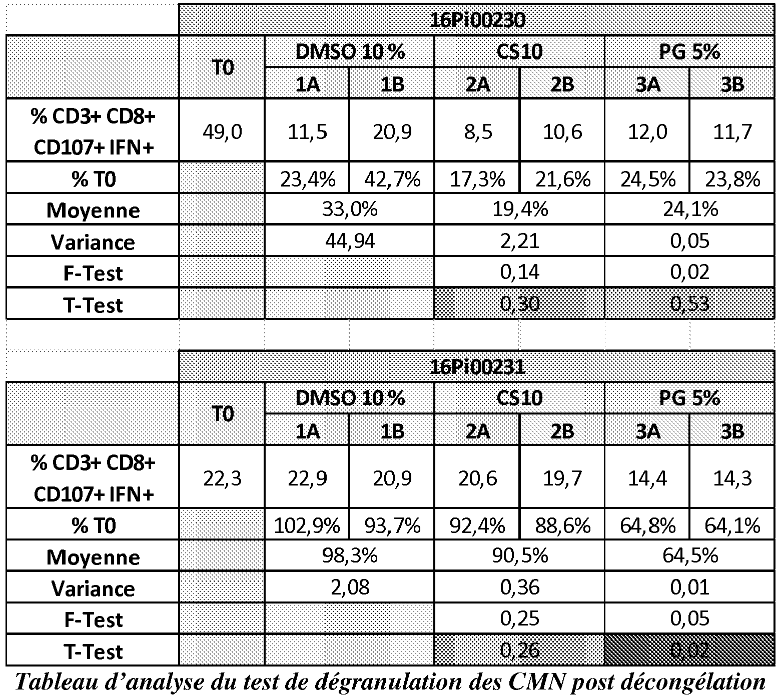

### Analyse du phénotype

Elle permet de déterminer la composition cellulaire des CMN donnée en pourcentages.

Parmi les cellules totales :
- Cellules hématopoïétiques (CD45+)
- Impuretés cellulaires non hématopoïétiques (CD45-)

Parmi les cellules CD45+ :
- lymphocytes T CD45+/3+
- lymphocytes T cytotoxique CD45+/3+/8+
- lymphocytes T helper CD45+/3+/4+
- NK (Natural Killer) CD45+/3+/16+ ^{et}/ₒᵤ 56+
- lymphocytes B CD45+/19+/3-
- monocytes CD45+/14+/3-

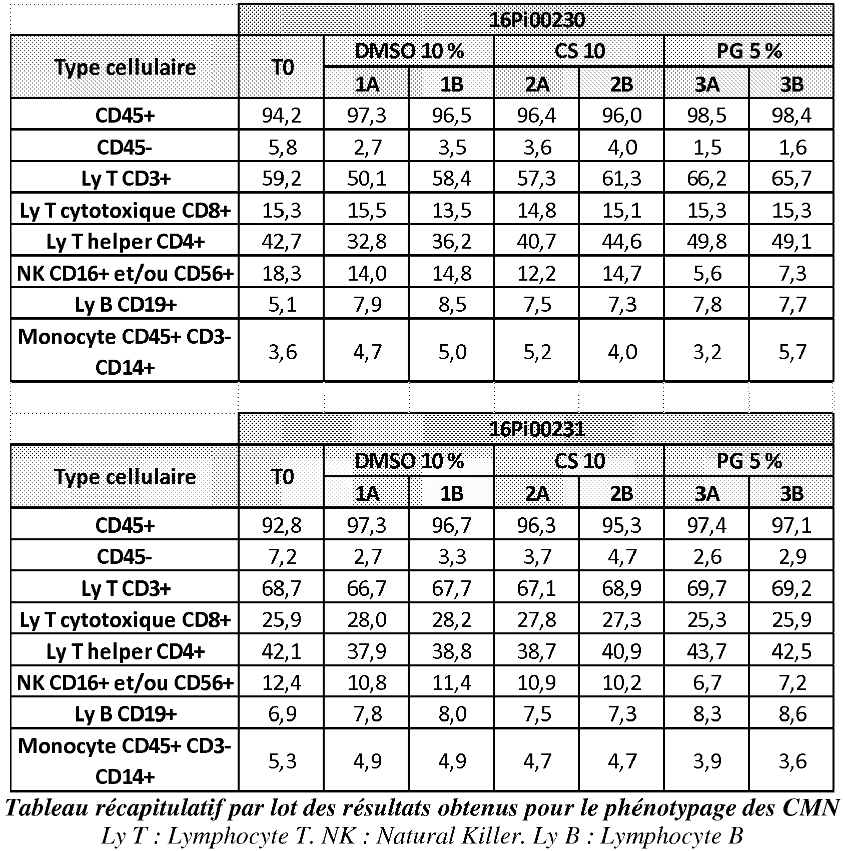

Les résultats obtenus pour chaque lot suivent la même tendance. En effet, les valeurs de TO sont proches et permettent une analyse des moyennes combinées des 2 lots. Le tableau ci-dessous présente ces valeurs en % du TO (n=2 réplicats sur 2 lots soit 4 valeurs en tout pour les conditions décongelées, TO fait en duplicat seulement).

**Tableau d'analyse des phénotypes Moyennes exprimées en % du T0 obtenues sur les 2 lots**

| **Type cellulaire** | **DMSO 10%** | **CS10** | **PG5%** |
|---|---|---|---|
| **CD45+** | 103,7% | 102,7% | 104,7% |
| **CD45-** | 47,4% | 61,8% | 32,0% |
| **Ly T CD3+** | 94,6% | 99,6% | 106,2% |
| **Ly T cytotoxique CD8+** | 101,7% | 102,0% | 99,6% |
| **Ly T helper CD4+** | 85,9% | 97,2% | 109,1% |
| **NK CD16+ et/ou CD56+** | 84,2% | 79,4% | 45,8% |
| **Ly B CD19+** | 138,2% | 126,4% | 137,3% |
| **Monocyte CD45+ CD3-CD14+** | 114,4% | 108,6% | 96,7% |

La formulation PG 5% permet une meilleure conservation des cellules CD45+ au détriment des cellules CD45- qui diminuent en proportion. En particulier, le pourcentage de lymphocytes T CD8+ reste constant et les CD4+ augmentent. Au contraire, les NK diminuent fortement. Le pourcentage de monocytes reste stable. C'est cette formulation qui se rapproche le plus des valeurs du TO et du CS10.

La conservation des NK est corrélée au pourcentage de DMSO dans les formulations. On observe un effet dose dépendant de la formulation PG5% sans DMSO (45,8%) à la formulation DMSO 10% (84,2%). La condition CS10 contenant 10% de DMSO est non significativement différente du DMSO 10%.

### Conclusion de l'étude

La présente étude de congélation a donc été réalisée sur 2 lots de CMN issus d'anneaux de cytaphérèses de 2 patients sains différents. Parmi les critères étudiés, les conclusions sont :
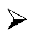 **Viabilité des cellules**. La cryo-préservation des CMN est efficace en formulation PG 5% (viabilité stable pour le PG5% par rapport à TO). Les résultats obtenus à 4h post décongélation sont satisfaisants et autorisent une utilisation en clinique.
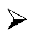 **Test de prolifération (CFSE)**. La congélation des CMN dans la formulation PG 5% n'altère pas la capacité de prolifération des cellules.
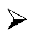 **Test de dégranulation**. La capacité immune de la population de lymphocytes T au sein des CMN est relativement variable et patient dépendant. Les résultats du premier lot, très variables, sont à relativiser (forte baisse de fonctionnalité dans tous les cas). Pour le 2^{e} lot, la condition PG 5% donne des résultats satisfaisants (baisse modérée par rapport à TO). Bien que les résultats obtenus post décongélation soient plus faibles que ceux obtenus pour le TO, les lymphocytes T conservent une capacité cytotoxique. La condition PG 5% est plus performante à préserver la fonctionnalité des cellules.
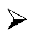 **Phénotype**. La formulation de congélation PG 5% montre une action spécifique : elle préserve préférentiellement certaines populations cellulaires. En effet, la formulation PG 5% est plus efficace à conserver les lymphocytes T (helper et cytotoxique) au contraire des NK.

### Exemple 3 : Tests avec la formulation selon l'invention pour cryopréserver des cellules souches mésenchymateuses (MSC)

La formulation suivante, nommée PG 5%, a été préparée :
Propylène glycol 5% + Coenzyme Q10 0,01% + Tréhalose 0,1M + sérum albumine humaine (AH) 4%.

Elle nécessite un temps d'incubation de 30 minutes maximum à 4°C avant congélation.

Cette formulation complexe est associée à un cycle de descente en température automatisé effectué au moyen d'un CRF (congélateur programmé) qui permet une descente en température complexe selon un cycle défini. Jusqu'à -40°C, les cellules sont considérées comme sensibles : la descente en température est lente et progressive pour permettre la formation de cristaux réguliers. Au-dessous de ce palier, le produit est considéré comme stable et la descente en température est rapide jusqu'à la température de stockage de -150°C maximum (azote gazeux ou liquide).

### Contexte de l'étude

Au cours de l'étude, 2 cycles ont été effectués, dont les caractéristiques sont présentées dans le tableau suivant :

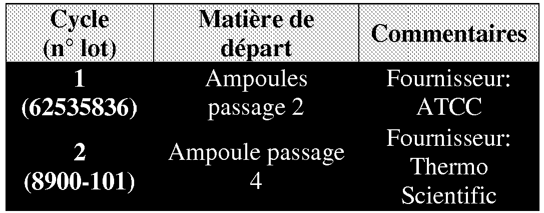

Chaque run correspond à un lot de cellules provenant d'un donneur différent. Pour ces 2 cycles, une ampoule de MSC provenant de l'ATCC pour le cycle 1 et une ampoule de Thermo Fisher Scientific pour le cycle 2 ont été décongelées. Les cellules étaient amplifiées durant plusieurs semaines puis congelées par CRF à la concentration de :
- 1,2 x 10⁶ cellules/mL pour le cycle 1 ;
- 2,5 x 10⁶ cellules/mL pour le cycle 2.

Afin de démontrer l'efficacité des formulations de congélation, 3 bras ont été réalisés. Pour chaque bras, 2 ampoules de 1mL ont été décongelées pour test.

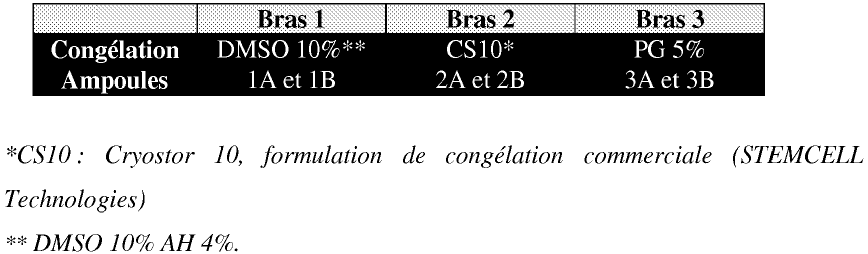

### Critère d'évaluation

Dans le but d'évaluer l'efficacité des formulations de congélation, différents tests, présentés dans le tableau suivant, ont été réalisés avant congélation (appelé TO) et après décongélation. Les résultats obtenus avec les cellules décongelées ont été comparés au TO et exprimé en % de TO.

| **T0** | **Post décongélation** | **Objectif** |
|---|---|---|
| Viabilité | Viabilité *post* décongélation (dans l'heure) | Critère général (critère primaire souvent libératoire qui autorise l'injection des cellules, souvent corrélé à l'efficacité et aux effets secondaires (impuretés acellulaires : débris, agrégats,...) |
| | Viabilité à 4h *post* décongélation | |
| Phénotype | | Caractérisation des sous-populations cellulaires et des cellules d'intérêt. En effet, le cycle de congélation/décongélation peut affecter spécifiquement certains sous types plus sensibles et modifier la balance du produit injecté (augmentation des impuretés cellulaires) |
| Test de prolifération | | Détermination du facteur d'amplification des cellules et donc de leur capacité de reprise corrélée à l'activité métabolique |

Les différents bras ont été comparés par une analyse statistique. La formulation de référence (bras 1) est le DMSO 10% + AH 4%.

Lors de l'analyse statistique, l'homogénéité des variances a été analysée par un test de Fisher (F-Test, variance homogène si p > 0,05). Un test d'égalité des espérances avec deux observations de variances égales ou inégales était réalisé. Les moyennes sont non significativement différentes si p > 0,05. Les moyennes sont significativement différente si p < 0,05.

### Résultats

### Analyse de la viabilité des cellules

L'analyse de la viabilité *post* décongélation démontre que les conditions testées sont non significativement différentes du TO pour tous les bras congelés (valeurs > 98% du TO). Les différences démontrées par l'analyse statistique sont dues à la variabilité du test.

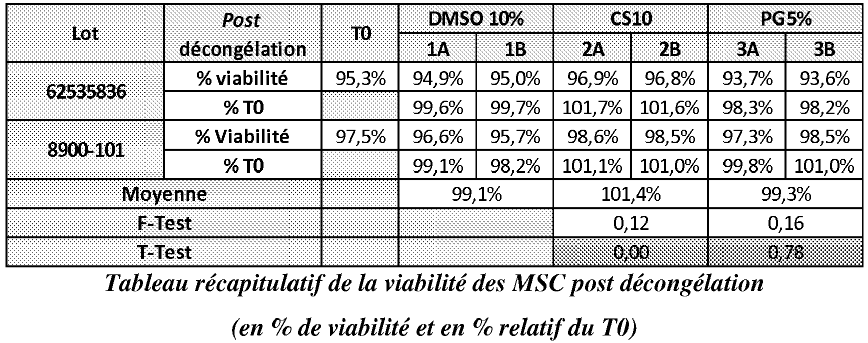

L'analyse de la viabilité des cellules après 4 heures à température ambiante dans leur contenant primaire et leur formulation de congélation permet d'évaluer la stabilité décongelée des cellules (simulation des conditions réelles de manipulation, transport et attente avant injection au patient). Elle met en évidence des pourcentages de viabilité stables pour les 3 bras.

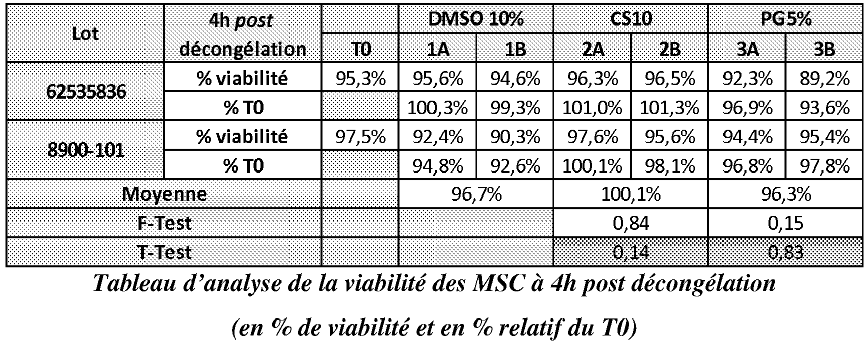

Pour les 2 lots, après 4h *post* décongélation, les variations sont faibles (entre 92 et 101% du TO). Après analyse comparative de la formulation PG 5%, aucune différence significative n'est démontrée par rapport à la référence (DMSO 10%) ainsi qu'au contrôle positif (CS10). La formulation a une capacité de conservation proche du CS10.

Dans le détail, la condition DSMO 10% est la plus variable entre les 2 lots de cellules testées tandis que la formulation CS10 est la plus stable.

Au final, les écarts sont faibles entre les différentes formulations donc la viabilité *post* décongélation n'est pas un critère discriminant.

### Analyse du phénotype

Un phénotype a été effectué afin de déterminer la stabilité de la population de MSC dans le produit décongelé. Pour les 2 lots, les pourcentages obtenus sont non significativement différents du T0. La quantité de cellules d'intérêt n'est donc pas affectée par le processus de congélation/décongélation.

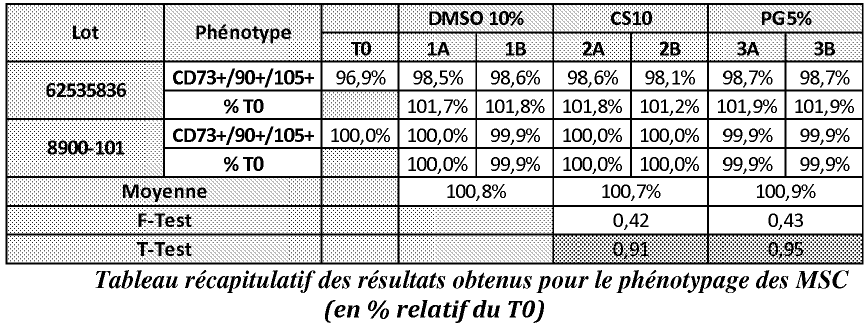

### Analyse de la prolifération des cellules

Le principe du test de prolifération consiste à ensemencer 50 000 cellules dans une plaque 6 puits et à compter le nombre de cellules obtenues après 7 jours en conditions de prolifération sans changement de milieu. La phase d'amplification des cellules a eu lieu dans le même milieu que celui de la phase de culture pré-congélation.

Pour les 2 cycles, les tests de prolifération ont échoués sur les conditions T0. Dans le premier cas, les cellules se sont décollées de manière inexpliquée (stress) et dans le 2^{e} cas, les cellules ont stagné. De ce fait, le bras formulé en PG 5% est comparé au bras contrôle (DMSO 10% et CS10).

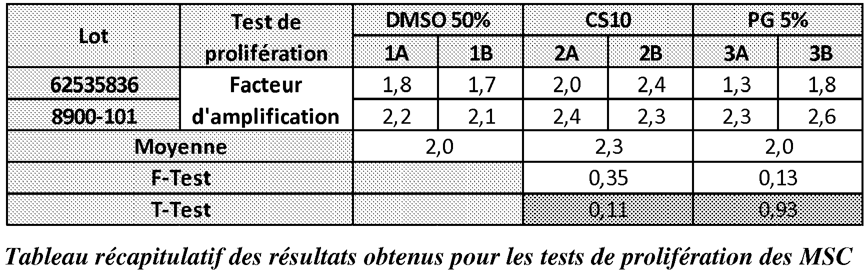

La capacité de prolifération des cellules est conservée pour chaque condition décongelée. L'analyse statistique ne démontre pas de différence significative entre eux. Par conséquent, la formulation PG 5% est équivalente aux références.

### Conclusion de l'étude

La présente étude réalisée sur 2 lots de MSC humaines issues d'ampoules commerciales remises en culture démontre que :
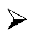 **Viabilité des cellules**. La cryo-préservation des MSC est efficace en formulation PG 5% en terme de viabilité cellulaire puisqu'elle est toujours supérieure à 98% du T0. De plus, elle est équivalente au CS10 (contrôle positif). Les résultats obtenus à *4h post* décongélation sont excellents (pas de diminution de la viabilité) et valident une utilisation clinique.
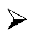 **Test de prolifération**. La congélation des MSC avec les différentes formulations n'affecte pas la capacité de prolifération des cellules. Cependant, en l'absence de T0, il n'est pas possible de la qualifier. Les facteurs d'amplification obtenus pour les cellules formulées en PG 5% ne sont pas différents des contrôles. Ceci démontre l'équivalence des formulations avec le CS10 (témoin contrôle).
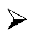 **Phénotype**. La formulation de congélation PG 5% préserve parfaitement le pourcentage de cellules d'intérêts (MSC).

Les 2 lots de MSC ont été cultivés de 2 façons différentes, le premier lot reçu de l'ATCC a été amplifié pendant 1 mois et demi dans les conditions recommandées par l'ATCC (milieu ATCC + kit de croissance), l'autre provenant de Thermo Fischer pendant 3 semaines en condition DMEM + faible concentration de glucose + sérum de veau foetal (SVF) 10%. Ces différences sont sans impact sur les résultats puisque les tendances sont les mêmes pour les 2 cycles.

### Exemple 4 : Tests avec la formulation selon l'invention pour cryopréserver des fibroblastes

La formulation suivante, nommée PG 5%, a été préparée :
Propylène glycol 5% + Coenzyme Q10 0,01% + Tréhalose 0,1M + sérum albumine humaine (AH) 4%.

Elle nécessite un temps d'incubation de 30 minutes maximum à 4°C avant congélation.

Cette formulation complexe est associée à un cycle de descente en température automatisé effectué au moyen d'un CRF (congélateur programmé) qui permet une descente en température complexe selon un cycle défini. Jusqu'à -40°C, les cellules sont considérées comme sensibles : la descente en température est lente et progressive pour permettre la formation de cristaux réguliers. Au-dessous de ce palier, le produit est considéré comme stable et la descente en température est rapide jusqu'à la température de stockage de -150°C maximum (azote gazeux ou liquide).

### Contexte de l'étude

Au cours de l'étude, 2 cycles ont été effectués, dont les caractéristiques sont présentées dans le tableau suivant :

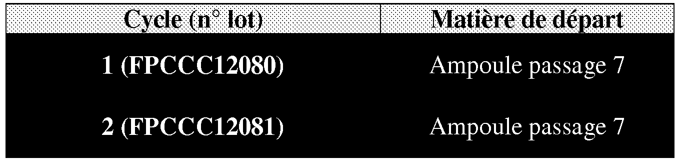

Pour ces 2 cycles, des ampoules de fibroblastes issus de patients sains différents ont été décongelées. Les cellules étaient amplifiées puis congelées à la concentration unique de **5 x 10⁶ cellules/mL**.

Afin de démontrer l'efficacité des formulations de congélation, 3 bras ont été réalisés. Pour chaque bras, 2 ampoules de 1mL ont été décongelées pour test.

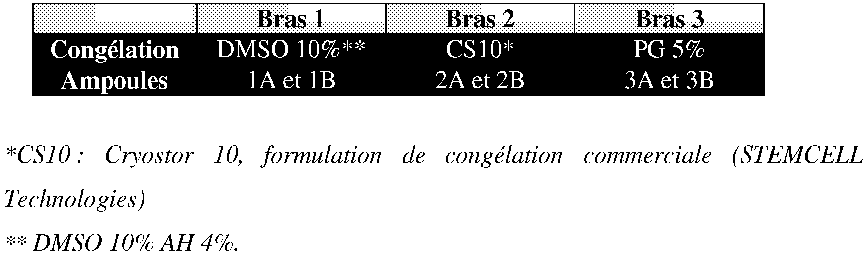

### Critère d'évaluation

Dans le but d'évaluer l'efficacité des formulations de congélation, différents tests, présentés dans le tableau suivant, ont été réalisés avant congélation (appelé TO) et après décongélation. Les résultats obtenus avec les cellules décongelées ont été comparés au TO et exprimé en % de TO.

| **T0** | **Post décongélation** | **Objectif** |
|---|---|---|
| Viabilité | Viabilité *post* décongélation (dans l'heure) | Critère général primaire souvent libératoire qui autorise l'injection des cellules, souvent corrélé à l'efficacité et aux effets secondaires (impuretés acellulaires : débris, agrégats,...) |
| | Viabilité à 4h *post* décongélation | |
| Phénotype | | Caractérisation des sous-populations cellulaires et des cellules d'intérêt. En effet, le cycle de congélation/décongélation peut affecter spécifiquement certains sous types plus sensibles et modifier la balance du produit injecté (augmentation des impuretés cellulaires) |
| Test de prolifération | | Détermination du facteur d'amplification des cellules et donc de leur capacité de reprise corrélée à l'activité métabolique/fonctionnelle |

Les différents bras ont été comparés par une analyse statistique. La formulation de référence (bras 1) est le DMSO 10% + AH 4%.

Lors de l'analyse statistique, l'homogénéité des variances a été effectuée par un test de Fisher (F-Test, variance homogène si p > 0,05). Un test d'égalité des espérances avec deux observations de variances égales était fait en cas de variance homogène. Dans le cas de variance non homogène, un test d'égalité des espérances à deux observations de variances différentes était réalisé. Les moyennes sont non significativement différentes si p > 0,05. Les moyennes sont significativement différentes si p < 0,05.

### Résultats

### Analyse de la viabilité des cellules

La viabilité *post* décongélation confirme l'efficacité de congélation sur tous les bras (>99% du TO).

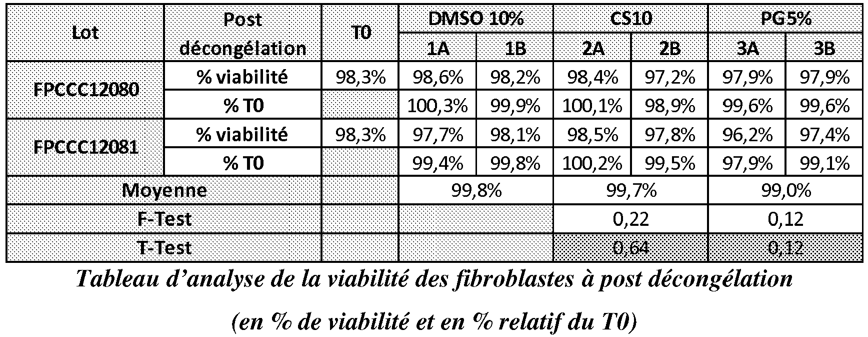

L'analyse de la viabilité des cellules après 4 heures à température ambiante dans leur contenant primaire permet d'évaluer la stabilité décongelée des cellules (simulation des conditions réelles de manipulation, transport et attente avant injection au patient).

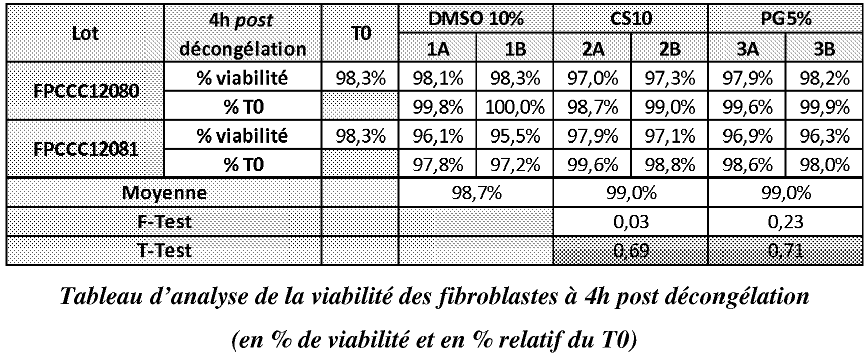

A 4h *post* décongélation, la viabilité des cellules reste stable (non significativement différente du TO) pour les 2 lots. Les formulations ne sont pas toxiques pour les cellules à température ambiante.

La formulation PG5% a le même potentiel de conservation que le DMSO 10% (référence) et le CS10 (contrôle positif), démontré par l'absence de différence significative lors de l'analyse statistique.

### Analyse du phénotype

Un phénotype a été effectué afin de déterminer la stabilité de la population de fibroblastes dans le produit. Les pourcentages retrouvés sont stables par rapport au TO. La quantité de cellules d'intérêt n'est donc pas affectée par le processus de congélation/décongélation.

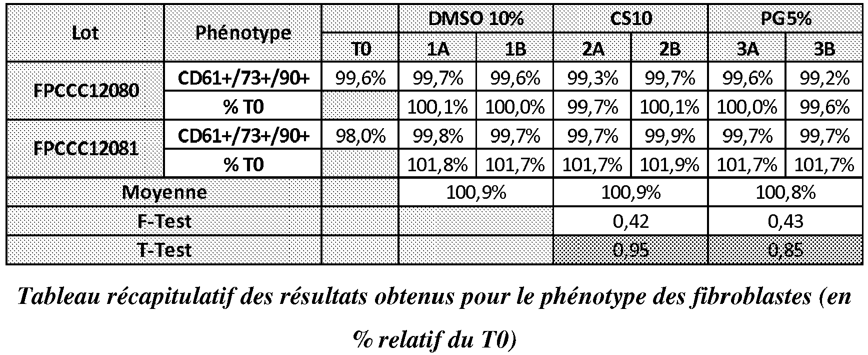

### Analyse de la prolifération des cellules

Le principe du test de prolifération consiste à ensemencer 50 000 cellules par puits dans une plaque 6 puits et à mesurer le nombre de cellules obtenues après 3 jours d'incubation.

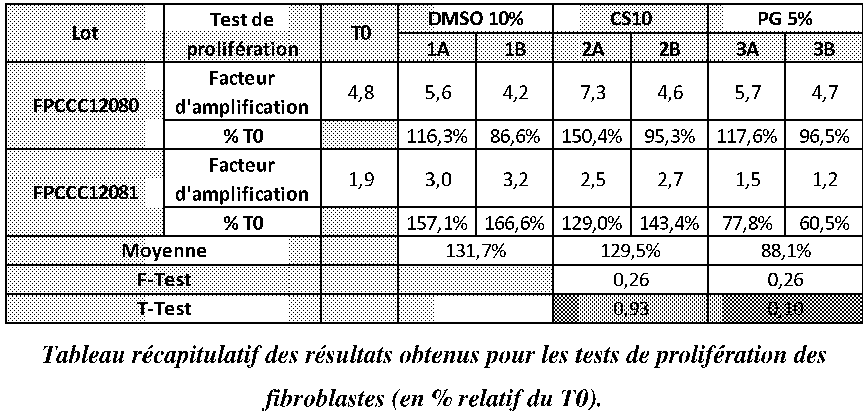

D'après les résultats obtenus, les cellules décongelées conservent leur capacité de prolifération et ne présentent pas de différence significative entre elles.

Cependant, une tendance à la baisse est observée sur le bras 3 (PG5%) en comparaison à la formulation de référence DMSO 10% et au T0, notamment sur le lot FPCCC12081 ou la valeur en % du TO moyen est de 69,2% contre 161,9% pour le DMSO 10%. Cette tendance n'est pas retrouvée au cours du premier cycle et est certainement patient dépendante.

### Conclusion de l'étude

La présente étude de congélation a donc été réalisée sur 2 lots de fibroblastes humains de donneurs différents. Parmi les critères étudiés, les conclusions sont :
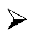 **Viabilité des cellules**. La cryo-préservation des fibroblastes est efficace en formulation PG 5% en terme de viabilité cellulaire puisqu'elle est toujours supérieure à 97% du T0. De plus, elle est équivalente au CS10 (contrôle positif). Les résultats obtenus à 4h *post* décongélation sont satisfaisants (pas de diminution observable de la viabilité) et permettent une utilisation clinique.
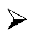 **Test de prolifération**. La capacité de prolifération des fibroblastes est dépendante du lot considéré. Pour le premier lot, les cellules ne sont pas affectées par la congélation, ce qui se traduit par des facteurs d'amplification supérieurs ou égaux au TO pour les 4 bras. Pour le 2^{e} cycle, les taux d'amplification sont meilleurs pour les cellules décongelées hormis la condition PG 5% (69,2% du TO).
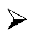 **Phénotype**. La formulation de congélation PG 5% préserve efficacement le pourcentage de cellules d'intérêts (fibroblastes).

## Revendications

1. Composition de cryoconservation comprenant, dans un milieu physiologiquement acceptable :
a) de l'albumine humaine,
b) au moins un saccharide choisi parmi les disaccharides et les trisaccharides,
c) du coenzyme Q10, et au moins un alcanediol en C3-C5, et
d) des cellules à visée thérapeutique, à l'exception de lymphocytes infiltrant la tumeur,
lesdits lymphocytes infiltrant la tumeur étant obtenus par culture *in vitro* de lymphocytes infiltrant la tumeur à partir de prélèvement de nodules cutanés en transit, ganglion ou métastase d'un patient atteint d'un mélanome de stade 3 ou 4, ladite culture comprenant l'émergence desdits lymphocytes infiltrant la tumeur contenus dans le prélèvement de nodules cutanés en transit, ganglion ou métastase, puis la stimulation des lymphocytes infiltrant la tumeur issus de l'étape d'émergence, puis enfin l'amplification des lymphocytes infiltrant la tumeur stimulés.

2. Composition selon la revendication 1, **caractérisée en ce que** le saccharide est choisi parmi les disaccharides.

3. Composition selon l'une des revendications 1 à 2, **caractérisée en ce que** le saccharide est un disaccharide choisi parmi le tréhalose et le saccharose.

4. Composition selon l'une des revendications 1 à 3, **caractérisée en ce que** l'albumine humaine est présente en quantité comprise entre 2 et 10% en poids par rapport au poids total de composition, de préférence entre 2.5 et 6% en poids.

5. Composition selon l'une des revendications 1 à 4, **caractérisée en ce que** le disaccharide est présent en concentration comprise entre 0.05M et 1M, de préférence entre 0.07M et 0.5M.

6. Composition selon l'une des revendications 1 à 5, **caractérisée en ce que** les cellules à visée thérapeutique sont choisies parmi les cellules NK, les monocytes, les lymphocytes B, les lymphocytes T, naturels ou génétiquement modifiés, tels que les lymphocytes T régulateurs, les lymphocytes T cytotoxiques, les lymphocytes T auxiliaires (ou helper) et les lymphocytes T ayant un récepteur antigénique chimérique (CAR), les myoblastes humains, les cellules souches hématopoïétiques, les cellules souches mésenchymateuses, les cellules cardiaques, les fibroblastes et toutes autres cellules naturelles ou génétiquement modifiées.

7. Composition selon l'une des revendications 1 à 6, **caractérisée en ce qu'**elle comprend :
a) de l'albumine humaine, de préférence en quantité comprise entre 2.5 et 6% en poids,
b) du tréhalose, de préférence en concentration comprise entre 0.07M et 0.5M,
c) du coenzyme Q10 et du propane-1,2-diol, de préférence respectivement en quantité comprise entre 0.007 et 0.1% en poids, et en quantité comprise entre 3 et 10% en volume, et
d) des cellules à visée thérapeutique, à l'exception de lymphocytes infiltrant la tumeur,
lesdits lymphocytes infiltrant la tumeur étant obtenus par culture *in vitro* de lymphocytes infiltrant la tumeur à partir de prélèvement de nodules cutanés en transit, ganglion ou métastase d'un patient atteint d'un mélanome de stade 3 ou 4, ladite culture comprenant l'émergence desdits lymphocytes infiltrant la tumeur contenus dans le prélèvement de nodules cutanés en transit, ganglion ou métastase, puis la stimulation des lymphocytes infiltrant la tumeur issus de l'étape d'émergence, puis enfin l'amplification des lymphocytes infiltrant la tumeur stimulés.

8. Procédé de cryoconservation d'au moins un échantillon de cellules à visée thérapeutique, à l'exception de lymphocytes infiltrant la tumeur, comprenant les étapes suivantes :
i) mélange de l'échantillon de cellules à visée thérapeutique avec:
a) de l'albumine humaine,
b) au moins un saccharide choisi parmi les disaccharides et les trisaccharides, et
c) du coenzyme Q10, et au moins un alcanediol en C3-C5, puis
ii) congélation du mélange obtenu à l'étape i),
lesdits lymphocytes infiltrant la tumeur étant obtenus par culture *in vitro* de lymphocytes infiltrant la tumeur à partir de prélèvement de nodules cutanés en transit, ganglion ou métastase d'un patient atteint d'un mélanome de stade 3 ou 4, ladite culture comprenant l'émergence desdits lymphocytes infiltrant la tumeur contenus dans le prélèvement de nodules cutanés en transit, ganglion ou métastase, puis la stimulation des lymphocytes infiltrant la tumeur issus de l'étape d'émergence, puis enfin l'amplification des lymphocytes infiltrant la tumeur stimulés.

9. Procédé de cryoconservation selon la revendication 8, **caractérisé en ce que** la congélation ii) est effectuée jusqu'à une température comprise entre -100°C et -180°C, de préférence comprise entre -140°C et -160°C.

10. Procédé de cryoconservation selon l'une des revendications 8 ou 9, **caractérisé en ce que** la congélation ii) est réalisée en plaçant le mélange obtenu en i) dans un récipient immergé dans un mélange d'isopropyl alcool à +4°C, le tout étant mis à une température comprise entre -70°C et -100°C.

11. Procédé de cryoconservation selon l'une des revendications 8 à 10, **caractérisé en ce que** la congélation ii) est effectuée à l'aide d'un congélateur programmé.

12. Utilisation d'une composition comprenant, dans un milieu physiologiquement acceptable :
a) de l'albumine humaine,
b) au moins un saccharide choisi parmi les disaccharides et les trisaccharides, et
c) du coenzyme Q10, et au moins un alcanediol en C3-C5, pour la cryoconservation d'au moins un échantillon de cellules à visée thérapeutique, à l'exception de lymphocytes infiltrant la tumeur,
lesdits lymphocytes infiltrant la tumeur étant obtenus par culture *in vitro* de lymphocytes infiltrant la tumeur à partir de prélèvement de nodules cutanés en transit, ganglion ou métastase d'un patient atteint d'un mélanome de stade 3 ou 4, ladite culture comprenant l'émergence desdits lymphocytes infiltrant la tumeur contenus dans le prélèvement de nodules cutanés en transit, ganglion ou métastase, puis la stimulation des lymphocytes infiltrant la tumeur issus de l'étape d'émergence, puis enfin l'amplification des lymphocytes infiltrant la tumeur stimulés.

## Patentansprüche

1. Kryokonservierungs-Zusammensetzung, umfassend in einem physiologisch akzeptablen Medium:
a) Humanalbumin,
b) wenigstens ein Saccharid, gewählt aus Disacchariden und Trisacchariden,
c) Coenzym Q10 und wenigstens ein C3-C5-Alkanediol, und
d) Zellen für therapeutische Zwecke, mit Ausnahme von tumorinfiltrierenden Lymphozyten, wobei die tumorinfiltrierenden Lymphozyten durch In-vitro-Kultur von tumorinfiltrierenden Lymphozyten erhalten werden, aus Proben von Hautknötchen im Übergang, Lymphknoten oder einer Metastase eines Patienten, der an einem Melanom der Stadien 3 oder 4 leidet, wobei die Kultur die Emergenz der tumorinfiltrierenden Lymphozyten umfasst, die in der Probe der Hautknötchen im Übergang, des Lymphknotens oder der Metastase enthalten sind, dann das Stimulieren der tumorinfiltrierenden Lymphozyten aus dem Emergenzschritt und schließlich das Verstärken der stimulierten tumorinfiltrierenden Lymphozyten.

2. Zusammensetzung nach Anspruch 1, **dadurch gekennzeichnet, dass** das Saccharid gewählt ist aus Disacchariden.

3. Zusammensetzung nach einem der Ansprüche 1 bis 2, **dadurch gekennzeichnet, dass** das Saccharid ein Disaccharid ist, gewählt aus Trehalose und Saccharose.

4. Zusammensetzung nach einem der Ansprüche 1 bis 3, **dadurch gekennzeichnet, dass** das Humanalbumin in einer Menge von 2 bis 10 Gew.-%, bezogen auf das Gesamtgewicht der Zusammensetzung, vorzugsweise von 2,5 bis 6 Gew.-% vorliegt.

5. Zusammensetzung nach einem der Ansprüche 1 bis 4, **dadurch gekennzeichnet, dass** das Disaccharid in einer Konzentration von 0,05M bis 1M, vorzugsweise von 0,07M bis 0,5M vorliegt.

6. Zusammensetzung nach einem der Ansprüche 1 bis 5, **dadurch gekennzeichnet, dass** die Zellen für therapeutische Zwecke gewählt sind aus Immunzellen, wie NK-Zellen, Monozyten, B-Lymphozyten, natürlichen oder genetisch veränderten T-Lymphozyten, wie regulatorische T-Lymphozyten, zytotoxische T-Lymphozyten, T-Helferzellen (oder 'Helpfer') und T-Lymphocyten mit einem chimären Antigenrezeptor (CAR), menschliche Myoblasten, hämatopoetische Stammzellen, mesenchymale Stammzellen, Herzzellen, Fibroblasten und alle anderen natürlichen oder genetisch veränderten Zellen.

7. Zusammensetzung nach einem der Ansprüche 1 bis 6, **dadurch gekennzeichnet, dass** sie umfasst:
a) Humanalbumin, vorzugsweise in einer Menge von 2,5 bis 6 Gew.-%,
b) Trehalose, vorzugsweise in einer Konzentration von 0,07M bis 0,5M,
c) Coenzym Q10 und Propan-1,2-diol, vorzugsweise jeweils in einer Menge von 0,007 bis 0,1 Gew.-% und in einer Menge von 3 bis 10 Vol.-%, und
d) Zellen für therapeutische Zwecke, mit Ausnahme von tumorinfiltrierenden Lymphozyten, wobei die tumorinfiltrierenden Lymphozyten durch In-vitro-Kultur von tumorinfiltrierenden Lymphozyten erhalten werden, aus Proben von Hautknötchen im Übergang, Lymphknoten oder einer Metastase eines Patienten, der an einem Melanom der Stadien 3 oder 4 leidet, wobei die Kultur die Emergenz der tumorinfiltrierenden Lymphozyten umfasst, die in der Probe der Hautknötchen im Übergang, des Lymphknotens oder der Metastase enthalten sind, dann das Stimulieren der tumorinfiltrierenden Lymphozyten aus dem Emergenzschritt und schließlich das Verstärken der stimulierten tumorinfiltrierenden Lymphozyten.

8. Verfahren der Kryokonservierung wenigstens einer Probe von Zellen für therapeutische Zwecke, mit Ausnahme von tumorinfiltrierenden Lymphozyten, umfassend die folgenden Schritte:
i) Mischen der Zellprobe für therapeutische Zwecke mit:
a) Humanalbumin,
b) wenigstens einem Saccharid, gewählt aus Disacchariden und Trisacchariden, und
c) Coenzym Q10 und wenigstens ein C3-C5-Alkanediol, dann
ii) Gefrieren der in Schritt i) erhaltenen Mischung,
wobei die tumorinfiltrierenden Lymphozyten durch In-vitro-Kultur von tumorinfiltrierenden Lymphozyten erhalten werden, aus Proben von Hautknötchen im Übergang, Lymphknoten oder einer Metastase eines Patienten, der an einem Melanom der Stadien 3 oder 4 leidet, wobei die Kultur die Emergenz der tumorinfiltrierenden Lymphozyten umfasst, die in der Probe der Hautknötchen im Übergang, des Lymphknotens oder der Metastase enthalten sind, dann das Stimulieren der tumorinfiltrierenden Lymphozyten aus dem Emergenzschritt und schließlich das Verstärken der stimulierten tumorinfiltrierenden Lymphozyten.

9. Verfahren der Kryokonservierung nach Anspruch 8, **dadurch gekennzeichnet, dass** das Gefrieren ii) bis zu einer Temperatur von -100°C bis -180°C, vorzugsweise von -140°C bis -160°C durchgeführt wird.

10. Verfahren der Kryokonservierung nach einem der Ansprüche 8 oder 9, **dadurch gekennzeichnet, dass** das Gefrieren ii) durch Einbringen der in i) erhaltenen Mischung in einen Behälter erfolgt, der in eine Mischung aus Isopropylalkohol bei +4°C eingetaucht wird, wobei das Ganze auf eine Temperatur von -70°C bis -100°C gebracht wird.

11. Verfahren der Kryokonservierung nach einem der Ansprüche 8 bis 10, **dadurch gekennzeichnet, dass** das Gefrieren ii) mittels eines programmierten Gefrierschranks durchgeführt wird.

12. Verwendung einer Zusammensetzung, die in einem physiologisch akzeptablen Medium umfasst:
a) Humanalbumin,
b) wenigstens ein Saccharid, gewählt aus Disacchariden und Trisacchariden, und
c) Coenzym Q10 und wenigstens ein C3-C5-Alkanediol, zur Kryokonservierung von wenigstens einer Zellprobe für therapeutische Zwecke, mit Ausnahme von tumorinfiltrierenden Lymphozyten, wobei die tumorinfiltrierenden Lymphozyten durch In-vitro-Kultur von tumorinfiltrierenden Lymphozyten erhalten werden, aus Proben von Hautknötchen im Übergang, Lymphknoten oder einer Metastase eines Patienten, der an einem Melanom der Stadien 3 oder 4 leidet, wobei die Kultur die Emergenz der tumorinfiltrierenden Lymphozyten umfasst, die in der Probe der Hautknötchen im Übergang, des Lymphknotens oder der Metastase enthalten sind, dann das Stimulieren der tumorinfiltrierenden Lymphozyten aus dem Emergenzschritt und schließlich das Verstärken der stimulierten tumorinfiltrierenden Lymphozyten.

## Claims

1. Cryopreservation composition comprising, in a physiologically acceptable medium:
a) human albumin,
b) at least one saccharide selected from disaccharides and trisaccharides,
c) coenzyme Q10, and at least one C3-C5 alkanediol, and
d) cells for therapeutic purposes, with the exception of tumour-infiltrating lymphocytes,
said tumour-infiltrating lymphocytes being obtained by *in vitro* culture of tumour-infiltrating lymphocytes from a sample of in-transit cutaneous nodules, lymph node or metastasis from a patient suffering from stage 3 or 4 melanoma, said culture comprising the emergence of said tumour-infiltrating lymphocytes contained in the sample of in-transit cutaneous nodules, lymph node or metastasis, followed by the stimulation of the tumour-infiltrating lymphocytes resulting from the emergence step, followed by finally the amplification of the stimulated tumour-infiltrating lymphocytes.

2. Composition according to claim 1, **characterised in that** the saccharide is selected from disaccharides.

3. Composition according to one of claims 1 to 2, **characterised in that** the saccharide is a disaccharide selected from trehalose and sucrose.

4. Composition according to one of claims 1 to 3, **characterised in that** the human albumin is present in a quantity between 2 and 10% by weight relative to the total composition weight, preferably between 2.5 and 6% by weight.

5. Composition according to one of claims 1 to 4, **characterised in that** the saccharide is present at a concentration between 0.05M and 1M, preferably between 0.07M and 0.5M.

6. Composition according to one of claims 1 to 5, **characterised in that** the cells for therapeutic purposes are selected from NK cells, monocytes, B lymphocytes, T lymphocytes, which are natural or genetically modified, such as regulatory T lymphocytes, cytotoxic T lymphocytes, helper T lymphocytes and T lymphocytes having a chimeric antigen receptor (CAR), human myoblasts, haematopoietic stem cells, mesenchymal stem cells, cardiac cells, fibroblasts and any other natural or genetically modified cells.

7. Composition according to one of claims 1 to 6, **characterised in that** it comprises:
a) human albumin, preferably in a quantity between 2.5 and 6% by weight,
b) trehalose, preferably at a concentration between 0.07M and 0.5M,
c) coenzyme Q10 and propane-1,2-diol, preferably respectively in a quantity between 0.007 and 0.1% by weight, and in a quantity between 3 and 10% by volume, and
d) cells for therapeutic purposes, with the exception of tumour-infiltrating lymphocytes,
said tumour-infiltrating lymphocytes being obtained by *in vitro* culture of tumour-infiltrating lymphocytes from a sample of in-transit cutaneous nodules, lymph node or metastasis from a patient suffering from stage 3 or 4 melanoma, said culture comprising the emergence of said tumour-infiltrating lymphocytes contained in the sample of in-transit cutaneous nodules, lymph node or metastasis, followed by the stimulation of the tumour-infiltrating lymphocytes resulting from the emergence step, followed by finally the amplification of the stimulated tumour-infiltrating lymphocytes.

8. Method for the cryopreservation of at least one sample of cells for therapeutic purposes, with the exception of tumour-infiltrating lymphocytes, comprising the following steps:
i) mixing the sample of cells for therapeutic purposes with:
a) human albumin,
b) at least one saccharide selected from disaccharides and trisaccharides, and
c) coenzyme Q10, and at least one C3-C5 alkanediol, then
ii) freezing the mixture obtained in step i),
said tumour-infiltrating lymphocytes being obtained by *in vitro* culture of tumour-infiltrating lymphocytes from a sample of in-transit cutaneous nodules, lymph node or metastasis from a patient suffering from stage 3 or 4 melanoma, said culture comprising the emergence of said tumour-infiltrating lymphocytes contained in the sample of in-transit cutaneous nodules, lymph node or metastasis, followed by the stimulation of the tumour-infiltrating lymphocytes resulting from the emergence step, followed by finally the amplification of the stimulated tumour-infiltrating lymphocytes.

9. Cryopreservation method according to claim 8, **characterised in that** the freezing ii) is performed to a temperature between -100°C and -180°C, preferably between - 140°C and -160°C.

10. Cryopreservation method according to one of claims 8 or 9, **characterised in that** the freezing ii) is carried out by placing the mixture obtained in i) in a container submerged in a mixture of isopropyl alcohol at +4°C, the whole being brought to a temperature between -70°C and -100°C.

11. Cryopreservation method according to one of claims 8 to 10, **characterised in that** the freezing ii) is performed using a programmed freezer.

12. Use of a composition comprising, in a physiologically acceptable medium:
a) human albumin,
b) at least one saccharide selected from disaccharides and trisaccharides, and
c) coenzyme Q10, and at least one C3-C5 alkanediol, for the cryopreservation of at least one sample of cells for therapeutic purposes, with the exception of tumour-infiltrating lymphocytes,
said tumour-infiltrating lymphocytes being obtained by *in vitro* culture of tumour-infiltrating lymphocytes from a sample of in-transit cutaneous nodules, lymph node or metastasis from a patient suffering from stage 3 or 4 melanoma, said culture comprising the emergence of said tumour-infiltrating lymphocytes contained in the sample of in-transit cutaneous nodules, lymph node or metastasis, followed by the stimulation of the tumour-infiltrating lymphocytes resulting from the emergence step, followed by finally the amplification of the stimulated tumour-infiltrating lymphocytes.
